(19) Europäisches Patentamt
European Patent Office
Office européen des brevets

(11) EP 0 796 915 A2

(12) # EUROPÄISCHE PATENTANMELDUNG

(43) Veröffentlichungstag: **24.09.1997 Patentblatt 1997/39**

(51) Int. Cl.[6]: **C12N 15/54**, C12N 9/10, C12P 19/26, C12N 15/76

(21) Anmeldenummer: **97104115.7**

(22) Anmeldetag: **12.03.1997**

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **22.03.1996 DE 19611252**
**25.06.1996 DE 19625269**

(71) Anmelder: **BAYER AG**
**51368 Leverkusen (DE)**

(72) Erfinder:
- **Crueger, Anneliese, Dr.**
  **40699 Erkrath (DE)**
- **Dellweg, Hans-Georg, Dr.**
  **42113 Wuppertal (DE)**
- **Lenz, Jürgen Georg, Dr.**
  **51373 Leverkusen (DE)**
- **Schröder, Werner, Dr.**
  **42113 Wuppertal (DE)**
- **Pape, Hermann, Prof. Dr.**
  **48158 Münster (DE)**
- **Goeke, Klaus, Dr.**
  **58708 Menden (DE)**
- **Schaper, Beate, Dr.**
  **48151 Münster (DE)**
- **Hemker, Michael**
  **48151 Münster (DE)**
- **Piepersberg, Wolfgang, Prof. Dr.**
  **42349 Wuppertal (DE)**
- **Distler, Jürgen, Dr.**
  **42107 Wuppertal (DE)**
- **Stratmann, Ansgar**
  **45527 Hattingen (DE)**

Bemerkungen:
Der Anmelder hat nachträglich ein Sequenzprotokoll eingereicht und erklärt, dass dieses keine neuen Angaben enthält.

(54) **Verfahren zur Herstellung sowie zur Verwendung von Acarviosyl-Transferase bei der Umwandlung von Acarbose-Homologen in Acarbose, zur Herstellung von Acarbose-Homologen**

(57) Die Erfindung betrifft die Acarviosyl-Transferase aus Actinomyceten, vorwiegend aus Actinoplanes sp. SE 50/110 und seinen Mutanten, ein Verfahren zur Isolierung, Reinigung und Charakterisierung des Enzyms, zur Isolierung und Charakterisierung des die Acarviosyl-Transferase codierenden acbD-Gens, die Expression der Acarviosyl-Transferase in einem heterologen Wirtsorganismus, die Verwendung der Acarviosyl-Transferase zur Umwandlung von Acarbose-Nebenkomponenten in Acarbose bzw. die Herstellung von Acarbose-Homologen, die Verwendung der Acarviosyl-Transferase in der Acarbose-Aufreinigung sowie die Herstellung von Produktionsmutanten mit reduzierter Nebenkomponentenbildung durch Inaktivierung des Acarviosyl-Transferase-Gens.

EP 0 796 915 A2

## Beschreibung

Die Erfindung betrifft die Acarviosyl-Transferase (AT) aus Actinomyceten, vorwiegend aus Actinoplanes sp. SE 50/13 oder SE 50/110 und seinen Mutanten, ein Verfahren zur Isolierung, Reinigung und Charakterisierung des Enzyms, zur Isolierung und Charakterisierung des die Acarviosyl-Transferase codierenden acbD-Gens, die Expression der Acarviosyl-Transferase in einem heterologen Wirtsorganismus, die Verwendung der Acarviosyl-Transferase zur Umwandlung von Acarbose-Nebenkomponenten in Acarbose bzw. die Herstellung von Acarbose-Homologen, die Verwendung der Acarviosyl-Transferase in der Acarbose-Aufreinigung sowie die Herstellung von Produktionsmutanten mit reduzierter Nebenkomponentenbildung durch Inaktivierung der Acarviosyl-Transferase.

Gegenstand älterer Patentanmeldungen (z.B. DE 20 64 092, DE 22 09 834) ist die Erkenntnis, daß eine Reihe von Actinomyceten, vor allem die Actinoplanaceen, oligosaccharidartige Inhibitoren von Glycosidhydrolasen vorzugsweise kohlenhydratspaltender Enzyme des Verdauungstraktes bilden. Die Inhibitoren bestehen aus einer Acarviosyl-Einheit, die $\alpha$-1,4-glycosidisch mit Maltooligosacchariden oder anderen Zuckern verknüpft ist. Das Acarviosyl-Core kann zu beiden Seiten mit einer unterschiedlichen Anzahl von Glucoseeinheiten verknüpft sein. Die Anzahl der Glucosereste am Core bestimmt die spezifische Aktivität des Inhibitors. Kürzere Moleküle (Komponenten mit 1 - 5 Glucose-Einheiten) wirken vorwiegend auf Disaccharidasen, während mit zunehmender Anzahl der Glucopyranosen die Wirkung auf $\alpha$-Amylasen effektiver wird. Als potentester $\alpha$-Glucosidase-Inhibitor dieser Gruppe ist die Verbindung O-4,6-Dideoxy-4-[[1S-(1S,4R,5S,6S)-4,5,6-trihydroxy-3-(hydroxymethyl)-2-cyclohexen-1-yl]-amino]-D-glucopyranosyl-(14)-O-D-glucopyranosyl (14)-D-glucopyranose als Acarbose beschrieben [DE 23 47 782].

Acarbose wird bei der Bekämpfung des Diabetes mellitus in der Humanmedizin eingesetzt. Die Bildung des Sekundärmetaboliten Acarbose erfolgt durch Actinoplanes sp. SE 50 (CBS-Nr. 961.70) und durch eine natürliche Variante dieses Stammes, SE 50/110 (CBS 674.73) oder auch SE 50/13 (CBS 614.71) [DE 22 09 83] sowie durch deren Selektanten und Mutanten. In den genannten Patentanmeldungen, z.B. in den Beispielen 1 bis 4 der genannten deutschen Patentanmeldung P 20 09 834, wird die Gewinnung eines derartigen Saccharase-Inhibitors beschrieben. Neben Acarbose als Hauptprodukt treten dabei Acarviosylhaltige Verbindungen mit unterschiedlichen Maltooligosaccharid- und Disaccharidresten als Nebenkomponenten auf.

Acarbose besteht aus einem Acarviosylrest, der bei der Biosynthese nach derzeitigem Stand des Wissens als erstes gebildet wird, um dann mit einem Maltosylrest verknüpft zu werden. Im Zuge der Arbeiten zur Aufklärung der Acarbose-Biosynthese wurde von Goeke 1986 (Goeke, K., Enzymatische Untersuchungen zum Zuckerstoffwechsel und zur Biosynthese des $\alpha$-Glucosidase-Inhibitors Acarbose bei *Actinoplanes* spec.; Dissertation, Universität Münster) ein enzymatischer Austausch des Maltosylrests der Acarbose (Acarviosyl-Maltose) gegen radioaktiv markierte Maltose beschrieben: Bei Verwendung von [U-$^{14}$C]-Maltose entsteht eine im Maltosylrest radioaktiv markierte Acarbose. Das beteiligte Enzym, die Acarviosyl-Transferase (AT), wurde zunächst als Pseudodisaccharidyl(PDS)-Transferase bezeichnet. Aus der Tatsache, daß nach Zellaufschluß und differentieller Zentrifugation die Aktivität der Pelletfrakion um den Faktor 3.25 über der Acarbose-Maltose-Austauschreaktion der Überstand-Fraktion lag, wurde der Schluß gezogen, daß die PDS-Transferase membrangebunden ist. Von Schaper 1991 (Schaper, B., Biochemische und physiologische Studien zur Biosynthese des $\alpha$-Glucosidase-Inhibitors Acarbose; Dissertation, Universität Münster) wurde dieser Befund bestätigt und ein detaillierter Aufarbeitungsgang für das membrangebundene Enzym ausgearbeitet. Für das partiell gereinigte Enzym wurde ein pH-Optimum von 4.5 angegeben bei einem Temperatur-Optimum von 30°C und $Mn^{2+}$ als Cofaktor.

Überraschend wurde nun gefunden, daß die Acarviosyl-Transferase mit der Fähigkeit zum Austausch des Acarbose-Maltosylrests nicht membrangebunden vorliegt, sondern daß sich die AT vorwiegend im Kulturfiltrat findet, wobei die Enzymaktivität parallel zum Zellwachstum ansteigt. Aus dem Zellüberstand konnte das Enzym mit großer Reinheit isoliert werden. Dazu wurde das Enzym aus dein Kulturüberstand mit Ammoniumsulfat gefällt. Nach dem Zentrifugieren wurde das Sediment in Puffer (Glycerin und $CaCl_2$ enthaltend) gelöst, wieder zentrifugiert und der resultierende Überstand über eine Anionenaustauschersäule gegeben. Der Durchbruch enthielt die Acarviosyl-Transferase. Aus dieser Lösung konnte die AT durch Dialyse als partiell angereichertes Präparat erhalten oder durch Chromatographie an DEAE-Fractogel®, zweimalige Fällung mit sedimentierbarer Stärke und Desorption mit Acarbose oder Maltose gereinigt werden.

Die gereinigte Acarviosyl-Transferase hat ein MG von 76 kDa (SDS-PAGE) und ein Temperaturoptimum von 20 - 40°C. Das Enzym ist temperaturstabil bis ca. 40°C. Ein pH-Optimum von pH 6,2 - 6,9 wurde bestimmt sowie eine Abhängigkeit von $Ca^{2+}$-Ionen.

Das gereinigte Enzym wurde sequenziert. Die sich ergebende Basensequenz zeigt überraschenderweise eine gute Übereinstimmung mit der entsprechenden DNA-Sequenz des acb*D*-Gens aus dem Acarbose-Gencluster des Produktionsorganismus Actinoplanes sp. SE 50/110.

Überraschend ist weiterhin, daß die Acarviosyl-Transferase den Maltosylrest der Acarbose gegen andere Zuckerreste unter Bildung von Acarbose-Homologen austauschen kann bzw. durch Austausch der Zuckerreste bei den während der Fermentation gebildeten Acarbose-ähnlichen Nebenkomponenten gegen einen Maltoserest zur Acarbose-Bildung befähigt ist. Dabei katalysiert die Acarviosyl-Transferase die allgemeine Reaktion

Acarviosyl-X + Y → Acarviosyl-Y + X

(X = Glucose, Maltose, Maltooligisaccharide u.a. Zucker,
Y = Glucose, Maltose, Maltooligosaccharide u.a. Zucker).

Die Erfindung offenbart daher:

Das vollständige Verfahren zur Isolierung und Reinigung der Acarviosyl-Transferase aus Kulturen von Actinoplanes sp. SE 50/110 oder dessen Mutanten.

Die Charakterisierung der gereinigten Acarviosyl-Transferase.

Die Aminosäuresequenz, die nach tryptischer Spaltung des Enzyms über mehr als 100 Aminosäuren bestimmt wurde. Die sich daraus ergebende Basensequenz zeigt eine gute Übereinstimmung mit der Basensequenz des acbD-Gens.

Ein Verfahren zur Herstellung von Acarbose aus Acarbose-Nebenkomponenten durch Austausch der jeweiligen Zuckerreste gegen Maltose.

Ein Verfahren zur Herstellung von Acarbose-Homologen mit veränderten pharmakologischen Eigenschaften durch Austausch des Maltosylrests der Acarbose gegen geeignete andere Zuckerreste.

Die Verwendung von Acarviosyl-Transferase oder immobilisierter AT zur Isolierung von Acarbose aus der Kulturbrühe (Affinitätschromatographie) bei gleichzeitiger Umwandlung von Acarbose-Homologen in Acarbose.

Isolierung und Charakterisierung des die Acarviosyl-Transferase kodierenden acbD-Gens.

Die gentechnische Herstellung von Acarviosyl-Transferase in einem heterologen Wirtsorganismus.

Die Herstellung von verbesserten Produktionsmutanten, wobei eine Eingrenzung des Produktspektrums in Actinoplanes auf Acarbose als das gewünschte Hauptprodukt der Biosynthese durch Ausschaltung der unerwünschten Nebenkomponentenbildung durch Inaktivierung des acbD-Gens erreicht wird.

Die Erfindung wird im folgenden detailliert beschrieben. Ferner wird die Erfindung durch den Inhalt der Ansprüche bestimmt.

Die Erfindung wird im folgenden detailliert beschrieben.

## I. Enzym-Aufreinigung

Kulturfiltrat einer zweiphasigen Anzucht von *Actinoplanes* sp. SE 50/110 oder seiner Mutanten:

| | | |
|---|---|---|
| Vorkultur: | Sojamehl entfettet | 2 % |
| | Glycerin | 2 % |
| | $CaCO_3$ | 0.2 % |
| | Leitungswasser | |
| | pH 7.2, Einstellung mit NaOH | |
| | 1000 ml Erlenmeyerkolben, 125 ml Füllvolumen | |
| | Inokulum: 5 ml Dauerkultur (72 h Vorkultur, Lagerung bei 20°C) | |
| | Inkubation: 72 h bei 30°C, 260 Upm | |
| Hauptkultur: | Sojamehl entfettet | 1 % |
| | Stärke | 3 % |
| | $CaCO_3$ | 0.2 % |
| | Leitungswasser | |
| | 1000 ml Erlenmeyerkolben, 125 ml Füllvolumen | |
| | Inokulum: 5 ml Vorkultur | |
| | Inkubation: 96-144 h bei 30°C, 260 Upm. | |

Nach einer Kulturdauer von 120 h wird das Maximum der AT-Aktivität mit 2.6 nkat/ml Kulturfiltrat erreicht. Dabei ist die AT das mengenmäßig vorherrschende Protein im Kulturfiltrat.

Das Aufreinigungsschema ist in <u>Tabelle 1</u> dargestellt.

**Tabelle 1** Aufreinigungsschema der Acarviosyl-Transferase

Die folgenden Puffer wurden verwendet:

Puffer 1: 25 mM Tris/HCl pH 8.5 + 10 % Glycerin + 1 mM $CaCl_2$
Puffer 2: 25 mM Tris/HCl pH 7.5 + 1 mM $CaCl_2$
Puffer 3: 10 mM Tris/HCl pH 7.5 + 1 mM $CaCl_2$
Puffer 4: 0.1 mM Tris/HCl pH 7.2 + 0.01 mM $CaCl_2$

Stärke*: aufgekochte, lösliche Stärke 12 h bei 4°C ("Kältefällung"), 60 min Zentrifugation bei 40.000 x g; Sediment eingesetzt

**Sojamehl-Stärke-Kultur**

↓ 3.000 x g; 10 min

**Kulturfiltrat**

↓ fraktionierte $(NH_4)_2SO_4$-Fällung (20 - 40 % Sättigung)
25.000 x g; 30 min (2 x)

**Sediment**

↓ Solubilisierung in Puffer 1
25.000 x g; 30 min

**Überstand**

↓ DEAE-Anionenaustauschersäule; 0 - 1 M NaCl

**Durchbruch**

↓ Dialyse (12 h), Puffer 1

**Retentat**

↓ DEAE-Anionenaustauschersäule; 0 - 1 M NaCl

**Fraktionen 0.15 - 0.35 M NaCl**

↓ Inkubation mit Stärke* (12 h)
40.000 x g; 60 min

**Sediment (in Puffer 2)**

↓ Inkubation mit Acarbose (25 mM; 2 h bei RT)
40.000 x g; 60 min

**Überstand**

↓ Dialyse (3 x 6 h) mit Puffer 3
Inkubation mit Stärke* (12 h); 40.000 x g; 60 min

**Sediment (in Puffer 3)**

↓ Inkubation mit Maltose (250 mM; 12 h)
40.000 x g; 60 min

**Überstand**

↓ Dialyse (3 x 6 h) mit Puffer 4

**gereinigte AT**

Die Bilanzierung der Aufreinigung ist Tabelle 2 zu entnehmen.

Tab. 2

| Aufreinigung der Acarviosyl-Transferase, Bilanzierung (zur Probenbezeichnung s. Aufreinigungsschema, Tab. 1) | | | | | |
|---|---|---|---|---|---|
| **Reinigungsstufe** | **Gesamtprotein (mg)** | **Gesamtaktivität (nkat)** | **spez. Aktivität (nkat/mg)** | **Ausbeute (%)** | **Anreicherung** |
| Kulturfiltrat | **400** | **1740** | **4,35** | **100** | **1** |
| 20 - 40 % $(NH_4)_2SO_4$ | **139** | **1147** | **7,75** | **66** | **1,8** |
| Durchbruch 1. Säule | **89** | **1078** | **12,1** | **62** | **2,8** |
| aktive Fraktion 2. Säule | **20** | **789** | **38,8 (43,1)*** | **45** | **8,9** |
| Stärke-Fällung + Acarbose-Ablösung | **9,7** | **538** | **55,5** | **31** | **12,8** |
| Stäke-Fällung + Maltose-Ablösung | **5,5** | **425** | **77,3** | **24** | **17,8** |

* aktivste Fraktion

Die Aufreinigung der AT ergibt eine 17,8 fache Anreicherung bei einer 24 %igen Ausbeute.

**II. Bestimmung der Acarviosyl-Transferase-Aktivität**

1. Radioaktivitätstest

a) Acarbose oder Acarbose-Homologe wurden mit $[^{14}C]$-Maltose in einem Tris-Maleinat-Puffer (pH 6,3) in Gegenwart von Acarviosyl-Transferasehaltigen Präparationen bei 30°C inkubiert. Anschließend wurde die Acarbose durch ein Kationenaustauscherharz von der Maltose getrennt. Die Höhe der $[^{14}C]$-Radioaktivität in der Acarbose-Fraktion im Verhältnis zur Gesamtradioaktivität ergibt die Austauschrate, die mit der AT-Aktivität korreliert ist.

Acarviosyl-($\alpha$-1,4)-(Zucker) + $[^{14}C]$-Maltose $\rightarrow$ Acarviosyl-($\alpha$-1,4)-$[^{14}C]$- Maltose + Zucker

b) $[^{14}C]$-Acarbose (in der Maltoseeinheit markiert) wurde mit Maltooligosacchariden oder anderen Zuckern in Gegenwart von AT inkubiert, wie unter a). Der Ansatz wurde - wie unter a) angegeben - behandelt und ausgewertet.

Acarviosyl-($\alpha$-1,4)-$[^{14}C]$-Maltose + Zucker $\rightarrow$ Acarviosyl-Zucker + $[^{14}C]$- Maltose

2. Dünnschichtchromatographie

Acarbose oder Acarbose-Homologe wurden mit Maltose, Maltooligosacchariden oder anderen Zuckern - wie unter a) angegeben - inkubiert. Der Reaktionsansatz enthielt:

10 $\mu$l AT-Präparation (AT in 0.1 mM Tris/HCl pH 7.2 + 0.01 mM $CaCl_2$; 4.5 nkat/ml)
10 $\mu$l Acarbose (70 mM Stammlösung) oder Acarbose-Homologe (ca. 30 mM)
10 $\mu$l Substrat (70 mM Stammlösung) oder Maltose (600 mM)

**Probenaufbereitung**

30 $\mu$l Reaktionsansatz
$\downarrow$ 18 h; 30°C
Zugabe von 70 $\mu$l Ethanol

↓ 5 min Zentrifugation bei 7.000 g
Abnahme von 80 µl Überstand → 5 µl für die DC
↓
75 µl
↓ Trocknung im Vakuumkonzentrator
Probe für HPLC

DC: Kieselgel 60 DC-Alu-Folien (Merck), Laufmittel : Butanol : Ethanol : Wasser (50 : 30 : 20); Färbung: Cer-Sprühreagenz; Entwicklung bei 110° C.

3. HPLC-Test

Acarbose oder Acarbose-Homologe wurden mit Maltose, Maltooligosacchariden oder anderen Zuckern - wie unter 2. angegeben - inkubiert. Nach Fällen der Proteine wurde die verbleibende Lösung mittels HPLC-ECD bzw. HPLC-UV auf ihre chemische Zusammensetzung analysiert.

**III. Eigenschaften der Acarviosyl-Transferase**

| | |
|---|---|
| Molmasse | 76 kDA (SDS-PAGE) |
| pH-Optimum | 6.2 - 6.9 |
| Temperatur-Optimum | 30°C (20 - 40°C) |
| Temperaturstabilität | bis ca. 40°C |
| Spurenelementabhängigkeit | $Ca^{2+}$ |

Aufgrund der unten aufgelisteten Akzeptorspezifitäten läßt sich die folgende allgemeine Formel für das Akzeptormolekül ableiten

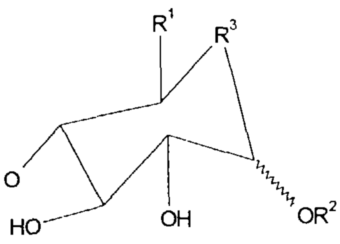

| | |
|---|---|
| $R^1$ | H, $CH_2OH$, $CH_3$ |
| $R^2$ | H, $(CH_2)_mCH_3$, m = 0 - 10 |
| | Pyranosen [α(1->2), (1->3), (1->4), (1->6), β(1->2), (1->3), (1->4)] |
| | Furanosen [α(1->6)] |
| | Glucit, Phenyl-, Nitrophenyl-, u.s.w. |
| $R^3$ | O, S, CHOH |
| Akzeptorspezifität | |
| | Cellobiose |
| | Deoxy-D-Glucose |
| | D-Gluconsäurelacton |
| | D-Glucose |
| | Isomaltose |
| | Isomaltotriose |
| | Laminaribiose (3-*O*-β-D-Glucopyranosyl-D-glucose) |
| | Maltose |
| | Maltotriose |
| | Maltotetraose |
| | Maltopentaose |
| | Maltohexaose |
| | Maltoheptaose |

| | |
|---|---|
| | Methyl-D-Glucopyranosid |
| | Palatinose |
| | Panose (6-α-Glycosyl-Maltose) |
| | Sophorose (2-*O*-β-D-Glucopyranosyl-α-D-glucose) |
| | Xylobiose |
| | L-Xylose |
| | D-Xylose |
| | Nigerose |
| | L(-)-Glucose |
| | 5-Thio-D-glucose |
| | myo-Inosit |
| | Maltitol |
| | Amygdalin |
| | Amylopektin |
| | Dextrin |
| | α-D(+)-Maltose-1-phosphat |
| | 4-Nitrophenyl-α-D-glucopyranosid |
| | 4-Nitrophenyl-β-D-xylopyranosid |
| | D(-)-Salicin |
| | Phenyl-α-D-glucopyranosid |
| | Octyl-D-glucopyranosid |
| | Nonyl-β-D-glucopyranosid |
| | Octyl-β-D-maltopyranosid |
| | Decyl-β-D-maltopyranosid |
| Donorspezifität | Acarbose |
| | Acarbose Nebenkomponente 2 |
| | Acarbose Nebenkomponente 4A |
| | Acarbose Nebenkomponente 4B |
| | Acarbose Nebenkomponente 4C |
| | Acarbose Nebenkomponente B |
| | Pseudoacarbose |

**IV. Proteinsequenzierung**

Die Analyse der N-terminalen Sequenz von Fragmenten der Acarviosyl-Transferase wurde mit dem Gas-Liquid-Solid-Phase Proteinsequencer 473A der Fa. Applied Biosystems (Foster City, CA., U.S.A.) durchgeführt. Dabei wurde das Standard-Sequenzierungs-programm benutzt. Das Gerät, die verwendeten Programme sowie das PTH-Trennsystem sind detailliert im zugehörigen Benutzerhandbuch beschrieben (User's manual protein sequencing system model 473A (1989), Applied Biosystems Foster City, CA 94404, U.S.A.).

Der Nachweis der PTH-Aminosäuren wurde on-line mit einer RP-18 Säule (220 mm x 2 mm, 5μ-Material) von Applied Biosytems durchgeführt. Die Identifizierung und quantitative Bestimmung der PTH-Aminosäuren erfolgte mit Hilfe eines 50 pM Standards aller PTH-Aminosäuren. Zur Datenauswertung wurde das Sequencer Data System 610A von Applied Biosystems benutzt. Die für den Protein Sequencer verwendeten Chemikalien wurden von Applied Biosystems bezogen.

Zur Trennung der tryptischen Peptide wurde das Smart-System der Fa. Pharmacia (D-Freiburg) eingesetzt.

Die HPLC-Säule (2.1 mm x 100 mm; 5μ-Material) zur Trennung der tryptischen Peptide wurde von Pharmacia (D-Freiburg) bezogen, Trypsin (Sequence Grade) von Boehringer Mannheim, alle übrigen Chemikalien von Merck (D-Darmstadt) oder Sigma (D-Deisenhofen).

**V. Isolierung und Sequenzierung des acbD-Gens** (AcbD Protein = AT)

Alle gentechnologischen Methoden wurden, wenn nicht anders angegeben, wie in Sambrook et al. (Molecular Cloning; A laboratory manual; 2nd edition, 1989; Cold Spring Harbour Laboratory Press, N.Y., U.S.A.) durchgeführt.

Die für das Screening verwandte Gensonde wurde aus dem Plasmid pAS2 (DE 195 07 214) isoliert. Das Plasmid pAS2 wurde aus E. coli DH5α mit Hilfe der boiling-Method oder durch alkalische Lyse präpariert und mittels der Restriktionsendonuklease BamHI hydrolysiert. Das resultierende 2,2 kb BamHI Fragment wurde isoliert und durch "nick-translation" mit $^{32}$P-markierten Desoxynukleotiden radioaktiv markiert. Dieses markierte Fragment wurde als Gensonde für die Isolierung von Acarbose-Biosynthese-Genen eingesetzt (DE 195 07 214) und wird im folgenden als acb-Sonde-II bezeichnet.

Acarbose-Biosynthese-Gene wurden wie folgt isoliert. Chromosomale DNA von Actinoplanes sp. SE 50/110 wurde mit dem Restriktionsenzym SstI hydrolysiert, gelchromatographisch getrennt und durch Southern-Hybridisierung mit der acbSonde-II auf das Vorhandensein einer homologen DNA-Sequenz untersucht. Das mit der Gensonde hybridisierte SstI-Fragment hatte eine Größe von 11 kb. Das 11 kb SstI-Fragment wurde aus dem Gel eluiert, in den Vektor pUC 18 ligiert und in E. coli DH5α kloniert. Das resultierende Plasmid bekam die Bezeichnung pAS5. Das Plasmid pAS5 wurde mit dem Restriktionsenzym PstI und HindIII hydrolysiert. Die dabei entstandenen Fragmente hatten folgende Größe:

1,4 kb PstI Fragment
5,4 kb PstI Fragment
0,05 kb PstI / HindIII Fragment
2,6 kb HindIII / PstI Fragment
3,8 kb PstI Fragment (1,1 kb PstI/SstI Fragment ligiert mit dem Vektor pUC 18)

Das 2,6 kb HindIII / PstI Fragment wurde aus dem Gel eluiert, in den Vektor pUC18 ligiert und in E. coli DH5α kloniert. Das resultierende Plasmid (pAS5/15.1) wurde einer Hydrolyse mit verschiedenen Restriktionsendonukleasen unterzogen und die entstandenen DNA-Fragmente in pUC18 in E. coli DH5α subkloniert und sequenziert. Um die DNA-Sequenz der aus pAS5/15.1 entstandenen Fragmente zu überprüfen, wurden zusätzlich mittels der PCR-Methode DNA-Fragmente aus chromosomaler DNA von Actinoplanes sp. amplifiziert, in pUC18 ligiert, in E. coli DH5α kloniert und anschließend sequenziert. Die DNA-Sequenzen der PCR-Primer wurden aus DNA-Sequenzen der aus pAS5/15.1 subklonierten Fragmente (siehe unten) abgeleitet.
Für die Bestimmung der DNA-Sequenz des 2,6 kb HindIII / PstI Fragments aus Actinoplanes sp. wurden folgende Plasmide konstruiert und die Sequenz der inserierten DNA jeweils bestimmt:

| | |
|---|---|
| pAS5/15.1 | = 2,6 kb HindIII / PstI Fragment aus pAS5 |
| pAS5/15.2 | = 0,75 kb SalI Fragment aus pAS5/15.1 |
| pAS5/15.3 | = 0,5 kb SalI Fragment aus pAS5/15.1 |
| pAS5/15.4 | = 0,4 kb SalI Fragment aus pAS5/15.1 |
| pAS5/15.5 | = 0,35 kb SalI Fragment aus pAS5/15.1 |
| pAS5/15.6 | = 1,25 kb PvuII Fragment aus pAS5/15.1 |
| pAS5/15.7 | = 0,7 kb PvuII / HindIII Fragment aus pAS5/15.1 |
| pAS5/15.9 | = 0,1 kb PvuII Fragment aus pAS5/15.1 |
| pAS5/15.12 | = 0,9 kb KpnI / NcoI Fragment aus pAS5/15.1 |
| pAS5/18 | = 0,3 kb PCR Fragment (Primer: s. Tab. 3) |
| pAS5/19 | = 0,3 kb PCR Fragment (Primer: s. Tab. 3) |

Für die DNA-Sequenzierung wurde die Methode von Sanger et al. (1977) oder ein davon abgeleitetes Verfahren angewandt. Es wurde mit dem Autoread Sequencing Kit (Pharmacia, D-Freiburg) in Verbindung mit dem Automated Laser Fluorescens (A.L.F.) DNA-Sequenzierungsgerät (Pharmacia, D-Freiburg) gearbeitet. Geeignete Fluorescein-markierte pUC "reverse sequencing" und "sequencing" Primer wurden käuflich erworben (Pharmacia, D-Freiburg).

Tabelle 3

| Sequenzen der Primer für die PCR und die Sequenzreaktion.<br>Primer für die PCR:<br>Plasmid pAS5/18: | |
|---|---|
| Primerbezeichnung | Sequenz |
| acbD3 | 5\`ACCAGGCCGAGGACGGCGCCC 3\` |
| acbD4 | 5\`AGCGGCATGTGCTTGACGGCG 3\` |
| Plasmid pAS5/19 | |
| Primerbezeichnung | Sequenz |
| acbD5 | 5\`ACCGGCTCGAACGGGCTGGCACC 3\` |
| acbD6 | 5\`CCCTCGACGGTGACGGTGGCG 3\` |
| Primer für die Sequenzreaktion: | |
| Primerbezeichnung | Sequenz |
| universal primer | 5\`GTAAAACGACGGCCAGT 3\` |
| reverse primer | 5\`GAAACAGCTATGACCATG 3\` |

**Beispiele**

**1. Herstellung, Reinigung und Charakterisierung der Acarviosyl-Transferase**

Der Wildstamm Actinoplanes sp. 50/110 oder eine davon abgeleitete Mutante wurde nach einer Vorkultur auf Sojamehl-Glycerin-Medium in der Produktionskultur auf Sojamehl-Stärke-Medium bei 30°C auf dem Rundschüttler mit einer Schüttelfrequenz von 260 Upm fermentiert. Nach einer Inkubationszeit von ca. 120 h wurde die Zellmasse abgetrennt. Aus dem Kulturüberstand wurde das Enzym mit Ammoniumsulfat (20 - 40 % Sättigung) gefällt. Nach dem Zentrifugieren wurde das Sediment in Puffer (25 mM Tris/HCl pH 8.5, Glycerin und $CaCl_2$ enthaltend) gelöst und wieder zentrifugiert. Der resultierende Überstand wurde über eine DEAE- Anionenaustauschersäule gegeben. Der Durchbruch enthielt die Acarviosyl-Transferase. Aus dieser Lösung konnte die AT durch Dialyse als partiell angereichertes Präparat erhalten oder durch Chromatographie an DEAE-Fractogel®, zweimalige Fällung mit Stärke und Desorption mit Acarbose oder Maltose gereinigt werden (s. Tab. 1). Bei einer Ausbeute von 24 % gelang eine Anreicherung um den Faktor 17,8.

Gemessen wurde die Aktivität der Acarviosyl-Transferase durch Übertragung des Acarviosylrests von Acarbose (Donor) auf Maltose (Akzeptor). Die Größe des Enzyms wurde nach SDS-PAGE mit 76.000 Da bestimmt, mit einem pH-Optimum bei pH 6,2 - 6,9 und einem Temperaturoptimum zwischen 20 - 40°C.

**2. Sequenzierung der Acarviosyl-Transferase**

Zur Bestimmung der internen Aminosäuresequenz wurde die Acarviosyl-Transferase mit Trypsin verdaut. Trypsin schneidet Proteine nach den Aminosäuren Lysin und Arginin.

Tryptische Spaltung der AT: Ca. 1 mg AT wurde in 1000 µl 6 M Guanidiniumchlorid / 0.5 M Tris-(hydroxymethyl)-aminomethan pH 8.6 gelöst. Nach Zusatz von 30 µl 1 M Dithiothreit (DDT) wurde die Probe bei 54°C über Nacht reduziert. Nach Zugabe von 60 µl 2 M Natriumiodacetat-Lösung wurde die Probe 30 min im Dunkeln inkubiert. Danach folgte eine Dialyse gegen 0.5 M Harnstoff / 0.1 M Ammonium-hydrogencarbonat (vollständiger Pufferaustausch nach 3 h und über Nacht; Dialyseschlauch mit 25 kD Ausschluß). Die so vorbehandelte Probe wurde unter Zusatz von 20 µg Trypsin (Sequence Grade) 18 h bei 37°C verdaut. Durch Trocknen in der Zentrifuge wurde die Probe auf ca. 100 µl aufkonzentriert.

HPLC-Trennung der tryptischen Peptide: Ein Drittel der Probe wurde auf eine RP-18-Säule (2.1 mm x 100 mm; 5 µ-Material) aufgetragen und unter Verwendung eines Smart-Systems getrennt (Lösung A 0.1 % TFA, Lösung B 0.1 % TFA / 60 % ACN; Detektion 215 nm, Fluß 0.15 ml/min, Raumtemperatur; Gradient: 7 min 0 % B, 52 min 70 %, 54 min 100 % B). Durch das hohe Molekulargewicht der AT entsteht beim Trypsin-Verdau ein sehr komplexes Gemisch. Rechromatographie einzelner Fraktionen ist daher Voraussetzung, um saubere Peptide für die spätere Sequenzierung zu erhalten.

Rechromatographie von Trennfraktionen: Die die Peptide enthaltenden Fraktionen wurden gepoolt und durch Trocknen in der Zentrifuge aufkonzentriert. Die Konzentrate wurden an einer RP-18-Säule (2.1 mm x 100 mm; 5 μ-Material) rechromatographiert (Lösung A 0.025 M $NH_4Ac$, Lösung B 0.025 M $NH_4Ac$ / 60 % ACN pH 6; Detektion 215 nm, Fluß 0.15 ml/min, Raumtemperatur; Gradient: 0 min 0 % B, 33 min 60 %, 38 min 100 % B). Nach Rechromatographie der Fraktionen 28 + 29 (Smar 4003) bzw. Fraktion 30 (Smar 4002) aus der ersten Trennung unter Verwendung verschiedener Chromatographie-Bedingungen war die Reinheit der erhaltenen Peptide ausreichend zur Sequenzierung der N-terminalen Sequenz.

Sequenzierung der N-terminalen Sequenz: Beispielsweise wurde Fraktion 32 der Rechromatographie (Smar 4002) durchTrocknen in der Zentrifuge eingedampft. Das Peptid wurde in TFA gelöst und zur Sequenzierung auf Glasfaserfilter aufgetragen, die zuvor mit BioBrene® behandelt waren. Das Peptid wurde unter Verwendung des Sequencer Zyklus "fast normal" sequenziert. Die PTH-Aminosäuren wurden mit Hilfe des 50 pmol PTH-Standards identifiziert und quantitativ bestimmt. Das Ergebnis der N-terminalen Sequenzanalyse ist in Tab. 4 dargestellt. Aus acht tryptischen Peptiden der AT wurden insgesamt 133 Aminosäuren analysiert.

**Tabelle 4** N-terminale Sequenzen der tryptischen Peptide der Acarviosyl-Transferase

1.1. Rechromatographie von Fraktion 28 + 29 (Smar4003)

1.2. Fraktion 35 der Rechromatographie

1
Asn-Leu-Gly-Val-Gly-Ala-Ile-Trp-Ile-Ser-Pro-His-Val-Asp-Asn-Ile-Asn-Val-Pro-
22 23
Ala-Ala-Gly-(Gly)...

2.1 Rechromatographie von Fraktion 30 (Smar4002)

2.2 Fraktion 32 der Rechromatographie

1
Thr-Gly-Lys-Pro-Val-Pro-Val-Gln-Phe-Thr-Val-Gln-Asn-Pro-Pro-Ala-Thr-Ala-Pro-
21
Gly-Glu...

2.3 Rechromatographie von Fraktion 25 (Smar4004)

2.3.1 Fraktion 31 der Rechromatographie

1 18
Ser-Thr-Val-Ala-Pro-Val-Leu-Gly-Ala-Gly-Gln-Val-Ala-Val-Trp-Ser-Tyr-Arg

2.3.2. Fraktion 25 + 26 der Rechromatographie

1
Tyr-Gln-Asp-Gln-Tyr-Tyr-Ser-Leu-Ala-Asp-Ile-Ala-Asp-Leu-Asp-Gln-Gln-Asn-
20
Pro-(Arg)

2.4 Rechromatographie von Fraktion 21 (Smar4005)

2.4.1. Fraktion 23 der Rechromatographie

1 12
Trp-Ile-Asn-Asp-Asp-Val-Tyr-Val-Tyr-Glu-Arg-Leu...

2.5 Rechromatographie der Fraktionen 31 + 32(Smar4001)

2.5.1 Fraktion 30 der Rechromatographie

1 18
Asp-Tyr-Leu-Tyr-Glu-Gln-Asp-Leu-Ile-Thr-Phe-Leu-Asp-Asn-Gln-Asp-Thr-Arg

2.6 Rechromatographie der Fraktionen 16 + 17 (Smar4007)

2.6.1 Fraktion 17 der Rechromatographie

1 9
Asp-Asp-Ala-Asn-Tyr-Trp-Met-Asp-Arg

2.7 Rechromatographie der Fraktion 20 (Smar4007)

2.7.1 Fraktion 11 der Rechromatographie

1 12
Ala-Val-Leu-Thr-Gly-Asn-Thr-Val-Tyr-Asp-Trp-Lys

**3. Umsetzung von Acarbose-Homologen zu Acarbose.**

Bei den Untersuchungen zur Donorspezifität der AT wurden in Versuchsansätzen Acarbose-Homologe, wie die Acarbose-Nebenkomponenten 2, 4A, 4B, 4C, Komponenten B und Pseudoacarbose

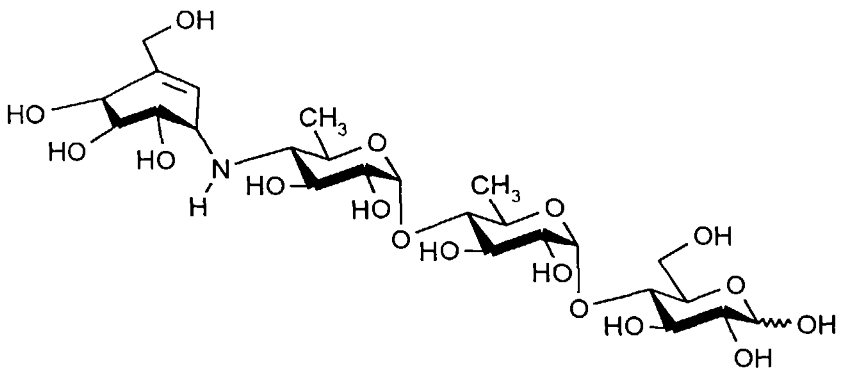

in Gegenwart von Acarviosyl-Transferase mit Maltose versetzt. Nach einer Reaktionszeit von 24 h bei 30°C wurden die Versuchsansätze mittels HPLC auf Aminophase mit UV Detektion analysiert. Die Auswertung (Tab. 5) zeigt, daß der Gehalt an Nebenkomponenten abnimmt, während der Acarbose-Gehalt zunimmt, d.h. von den Acarbose-Nebenkomponenten 2, 4A, 4B und 4C findet ein Transfer der Acarviosyleinheit auf Maltose statt.

Tabelle 5

| Umsetzung der Acarbose-Nebenkomponenten 2, 4A, 4B und 4C mittels AT zu Acarbose. | | | | | | |
|---|---|---|---|---|---|---|
| **Donoren** | **Reaktionsdauer (h)** | **HPLC- Analyse (g/l)** | | | | |
| | | **Acarbose** | **Komp. 2** | **Komp. 4A** | **Komp. 4B** | **Komp. 4C** |
| **Komp. 2** | **0** | **0.29** | **0.60** | | | |
| | **24** | **0.40** | **0.40** | | | |
| **Gemisch** | | | | | | |
| **Komp. 4A, Komp. 4B, Komp. 4C** | **0**<br>**24** | **0.53**<br>**1.70** | | **0.92**<br>**0.08** | **0.78**<br>**0.07** | **0.48**<br>**0.26** |

Mit Komponente B und Pseudoacarbose werden Spuren einer Umsetzung gefunden.

**4. Gewinnung von höheren Acarbose-Homologen aus Acarbose**

Bei den Untersuchungen zur Akzeptorspezifität der AT wurde Acarbose in Versuchsansätzen in Gegenwart ausreichend hoher Konzentrationen an Maltooligosacchariden u.a. Zuckern mittels Acarviosyl-Transferase umgesetzt. Nach einer Reaktionszeit von 18 h bei 30°C wurden die Versuchsansätze mittels HPLC auf Aminophase mit UV Detektion analysiert. Die Auswertung (Tab. 6) zeigt, daß neusynthetisierte Saccharide nachgewiesen werden, während gleichzeitig Maltose freigesetzt wird.

Tabelle 6

| Übertragung des Acarviosylrestes der Acarbose auf verschiedene Zucker als Akzeptoren ( FI = Flächenprozente; RT = Retentionszeit) | | | | | |
|---|---|---|---|---|---|
| **Akzeptoren** | **Acarbose (RT)** | **Akzeptor (RT)** | **Maltose (FI %)** | **neue Komponenten** | |
| | | | | **(FI %)** | **(RT)** |
| Aqua demin. | 31.4 | | 2.9 | 0 | |
| Cellobiose | 31.3 | 13.5 | 17.2 | 29 | 25.0 |
| Deoxy-D-Glucose | 30.4 | 4.5 | 2,6 | 1 | 6.6 |
| Gluconsäurelacton | 30.6 | 17.1 | 3.7 | 4 | 37.4 |
| D-Glucose | 30.2 | 5.7 | 7.6 | 11 | 18.0 |
| Isomaltose | 31.8 | 10.1 | 9.7 | 16 | 19.6 |
| Isomaltotriose | 30.9 | 13.9 | 5.4 | 7 | 23.5 |
| Laminaribiose | 28.5 | 15.6 | 16.3 | 27 | 30.1 |
| Maltose | 30.2 | 15.8 | 38.1 | | |
| Maltotriose | 31.8 | 26.8 | 19.8 | 21 | 38.8 |
| Maltotetraose | 31.1 | 38.1 | 19.9 | 20 | 40.6 |
| Maltopentaose | 32.6 | 40.6 | 17.9 | 20 | 42.1 |
| Maltohexaose | 31.2 | 41.7 | 17.2 | 15 | 43 |
| Maltoheptaose | 30.3 | 42.6 | 18.9 | 20 | 43.6 |
| Methyl-D-Glucopyranosid | 29.2 | 1.8 | 11.5 | 13 | 3.8 |
| Palatinose | 31.1 | 13.5 | 9.3 | 9 | 24.8 |
| Panose | 31.1 | 21.2 | 14 | 20 | 37.5 |
| Sophorose | 30.5 | 16.5 | 16.3 | 34 | 33.2 |
| Xylobiose | 28.5 | 10.8 | 20.8 | 22 / 5 | 16.7/17.3 |
| D-Xylose | 30.7 | 5.9 | 5.7 | 6 | 14.8 |
| L-Xylose | 29.1 | 5.8 | 11 | 15 | 16.0 |

Bei der Bestimmung der AT-Aktivität im Radioaktivitätstest wurde zusätzlich ein Austausch mit Dextrin beobachtet:

| Oligosaccharid | rel. Aktivität (%) |
|---|---|
| Maltose | 100 |
| Maltotriose | 27 |
| Maltotetraose | 40 |
| Maltoheptaose | 49 |
| Cellobiose | 15 |
| Dextrin | 45 |

**5. Modifiziertes Acarbose-Aufarbeitungsverfahren mittels AT:**

Die von der AT katalysierte Reaktion kann prinzipiell auch die Anreicherung von Acarbose aus Kulturlösungen mit gleichzeitiger Umwandlung von Acarbose-Homologen in Acarbose nach folgendem Prinzip gestatten:

1) Umsetzung von Acarbose oder Acarbose-Homologen [Acarviosyl-$(G)_n$] mit hochmolekularen Dextrinen oder Stärke [$(G)_m$] in Gegenwart von Acarviosyl-Transferase

$$\text{Acarviosyl-}(G)_n + (G)_m \rightarrow \text{Acarviosyl-}(G)_m + (G)_n$$

und Entfernen niedermolekularer Begleitstoffe durch Dialyse oder Fällen des Polysaccharids; anschließend

2) Umsetzung mit Maltose unter Freisetzung von Acarbose

$$\text{Acarviosyl-}(G)_m + \text{Maltose} \rightarrow \text{Acarbose} + (G)_m$$

Der gleiche Effekt läßt sich auch über einen Reaktor (z.B. Säule) mit Stärke und immobilisierter AT erreichen:

- Filtration roher Acarbose-Lösung über Stärke/AT-Säule
- Waschen zur Entfernung von Begleitstoffen
- Elution von Acarbose mit Maltose

In dem obigen Reaktionsschema stehen G für Glucose und m und n jeweils für eine ganze Zahl zwischen 1 und 20, wobei m und n unterschiedlich sind.

**6. Anzucht der E. coli Stämme, Präparation der Plasmid-DNA und Isolierung von DNA-Fragmenten**

E. coli DH5α wurde in LB-Medium bei 37°C bebrütet. Plasmidtragende Bakterien wurden unter Selektionsdruck (Ampicillin, 100 µg/ml) gehalten. Die Kultivierung erfolgte auf einem Rundschüttler bei 270 Upm . Als Übernachtkultur (ÜK) wurden Ansätze bezeichnet, die wenigstens 16 h bebrütet wurden.

Für die Präparation von Plasmid-DNA wurden die Zellen aus 1.5 ml einer unter Selektionsdruck bebrüteten ÜK eingesetzt. Die Isolierung der Plasmide erfolgte nach der Methode der alkalischen SDS-Lyse (Birnboim u. Doly, 1979).

Zur gezielten Hydrolyse von Vektor-DNA wurden ausschließlich Restriktionsendonukleasen nach Vorschrift des Herstellers (Gibco BRL, Eggenstein, Deutschland) eingesetzt. Zur Restriktion von 10 µg Plasmid-DNA wurden 5 U der jeweiligen Restriktionsendonuklease eingesetzt und 2 h bei 37°C inkubiert. Um eine vollständige Hydrolyse zu gewährleisten, wurde die gleiche Menge Restriktionsendonuklease ein zweites Mal zugegeben und erneut mindestens 1 h inkubiert.

Die gespaltene DNA wurde, je nach Größe der DNA-Fragmente, auf 0.5 - 1.2 %igen horizontalen Agarosegelen elektrophoretisch getrennt. Zur Elution wurde das Gelstück, welches das DNA-Fragment enthielt, mit einem sterilen Skalpell ausgeschnitten und gewogen. Die Elution der DNA-Fragmente aus der Agarose erfolgte mit dem JETsorb-Kit nach Vorschrift des Herstellers (Genomed, Bad Oeynhausen, Deutschland).

**7. Anzucht von Actinoplanes sp. SE 50/110, Präparation, Spaltung der chromosomalen DNA und gelelektrophoretische Trennung**

Actinoplanes sp. SE 50/110 wurde bei 30°C in TSB-Medium auf einem Rundschüttler für 3 d bebrütet. Die Vorkultur (5 ml) erfolgte bei 240 Upm in Kulturröhrchen, die Hauptkultur (50 ml) in 500 ml Schikanekolben bei 100 Upm. Die Zellen wurden nach der Kultivierung durch Zentrifugation sedimentiert und zweimal in TE-Puffer gewaschen.

Die Präparation der Gesamt-DNA erfolgte mit 1.5 - 2 mg Zellen (Frischgewicht) nach der Methode der Phenol/Chloroform-Extraktion (Hopwood et al., 1985). Die Hydrolyse der 20 µg chromosomaler DNA wurde mit 10 U des entsprechenden Restriktionsenzyms (Gibco BRL, Eggenstein, Deutschland) für 2 h bei 37°C in dem zugehörigen Puffer durchgeführt. Um eine vollständige Hydrolyse zu gewährleisten, wurde die gleiche Menge Restriktionsendonuklease ein zweites Mal zugegeben und erneut für mindestens 1 h inkubiert.

Die gespaltene DNA wurde auf 0.6 %igen horizontalen Agarosegelen elektrophoretisch getrennt.

Die Elution von DNA-Fragmenten erfolgte wiederum mit dem JETsorb-Kit (s. Beispiel 6).

**8. Herstellung der acb-Gensonde II**

Das nach Beispiel 6 präparierte Fragment aus pAS2 wurde mit dem "nick translation" System des Herstellers

Gibco BRL, Eggenstein, Deutschland, nach dessen Angaben radioaktiv markiert. Hierei wurden 0.5 - 1.0 µg DNA-Fragment eingesetzt. Es wurde [α $^{32}$P]dCTP eingesetzt (3000 Ci/mM; Amersham, Braunschweig, Deutschland). Anschließend wurde der Ansatz 10 Minuten gekocht (Denaturierung) und sofort zu der Hybridisieningslösung gegeben (s. Beispiel 9).

**9. DNA-Transfer auf Membranen, DNA-Hybridisierung (Southern-Hybridisierung) und Autoradiographie**

Die Übertragung von DNA-Fragmenten aus Agarosegelen auf Membranen erfolgte nach der Methode des Southern-Transfer (Southern, 1975). Die nach Beispiel 7 erhaltenen Agarosegele wurden 20 Minuten in 0.25 M HCl geschwenkt. Die Gele wurden auf drei Lagen Whatman-Papier 3MM (Whatman, Maidstone, England) gelegt und eine Hybond™-N+ Membran (Amersham, Braunschweig, Deutschland) luftblasenfrei aufgelegt. Darauf wurden mehrere Schichten saugfähiges Papier gelegt. Auf den Filterstapel wurde ein ca. 1 kg schweres Gewicht gestellt. Der DNA-Transfer erfolgte durch Durchsaugen von 0.4 M NaOH. Nach mindestens 12 h Transferzeit wurden die Nylonfilter mit 2 x SSC für 5 Minuten gespült und an der Luft getrocknet.

Die Nylon-Filter wurden dann mindestens 2 h bei 68°C in 50 - 100 ml Prähybridisierungslösung im Wasserbad geschüttelt. Dabei wurde die Lösung mindestens zweimal gewechselt. Die Hybridisierung fand im Hybridisierungsschrank für mindestens 12 h statt. Es wurden 15 ml Hybridisierungslösung, welche die acb-Sonde-II enthielt (s. Beispiel 8), eingesetzt.

Die Nylon-Filter wurden anschließend für jeweils 15 Minuten mit 6 x Postwash und 1 x Postwash gewaschen. Die Nylon-Filter wurden dann im noch feuchten Zustand mit Frischhaltefolie abgedeckt. Die Autoradiographie erfolgte mit Hyperfilm-MP (Amersham, Braunschweig, Deutschland) in einer lichtdichten Kassette mit Verstärkerfolien bei -80°C für mindestens 16 h.

**10. Isolierung und Klonierung von SstI -Fragmenten aus der Gesamt-DNA von Actinoplanes sp. SE 50/110.**

Chromosomale DNA aus Actinoplanes sp. wurde mit SstI vollständig hydrolysiert, durch Agarose-Gelelektrophorese getrennt und die DNA-Fragmente der Länge 9.0 - 12 kb aus der Agarose eluiert (s. Beispiel 6). Das Vektorplasmid pUC18 wurde aus E. coli DH5α präpariert, mit SstI hydrolysiert und mit alkalischer Phosphatase (Boehringer, Mannheim, Deutschland) nach Vorschrift des Herstellers behandelt. Die Ligation fand in einem Volumen von 20 µl statt, wobei das Verhältnis von Fragment zu Vektor 3 : 1 betrug mit 0.01 - 0.1 µg DNA im Ansatz. Es wurde 1 U der T4-DNA-Ligase mit dem entsprechenden Puffer (Gibco BRL, Eggenstein, Deutschland) eingesetzt.

Transformationskompetente Zellen von E. coli DH5α wurden mit vollständigen Ligationsansätzen transformiert (nach Hanahan, 1983). Ampicillin-resistente Transformanden wurden auf LB-Amp Selektivplatten (Ampicillin 100 µg/ml) übertragen.

**11. Identifizierung von Plasmiden, welche das 11 kb SstI-Fragment aus dem Acarbose-Biosynthesecluster enthalten**

Ampicillin-resistente Transformanden wurden auf das Vorhandensein des 11 kb-SstI-Fragments, welches mit der acb-Sonde-II hybridisiert, untersucht. Jeweils zehn dieser Klone wurden auf einer Selektivplatte ausgestrichen, über Nacht bebrütet und mit 3 ml LB-Medium von der Platte gewaschen. Es wurde dann aus 20 solcher Zehner-Pools die Plasmid-DNA isoliert (nach Birnboim u. Doly, 1979). Um die klonierten SstI-Fragmente aus dem Polylinker zu entfernen, wurden die 20 verschiedenen Plasmidpräparationen mit den Restriktionsendonukleasen EcoRI und HindIII hydrolysiert. Die Restriktionsansätze wurden dann auf einem 0.6 % Agarosegel elektrophoretisch getrennt und die DNA mittels Southern-Transfer aus dem Agarosegel auf einen Nylon-Filter übertragen (s. Beispiel 9). Die Hybridisierung erfolgte wiederum mit der acb-Sonde-II (s. Beispiel 9). Einer der Pools reagierte positiv mit der acb-Sonde-II und wurde in die zehn Einzelklone geteilt. Deren Plasmide wurden ebenfalls isoliert und der oben beschriebenen Methode unterworfen. Das hybridisierte Plasmid wurde mit pAS5 bezeichnet. Es enthält ein 10.65 kb SstI-Fragment.

**12. Klonierung des 2.6 kb HindIII / PstI -Fragments**

Um weitere Leserahmen identifizieren zu können, wurden - ausgehend von dem Plasmid pAS5 - mehrere HindIII / PstI -Subklone erstellt. Dafür wurde das Plasmid pAS5 mit den Restriktionsendonukleasen HindIII / und PstI hydrolysiert. Es entstanden folgende Fragmente:

1.4 kb PstI-Fragment
5.4 kb PstI-Fragment
0.05 kb PstI / HindIII-Fragment
2.6 kb HindIII / PstI-Fragment

3.8 kb PstI-Fragment (1.1 kb PstI / SstI-Fragment ligiert mit dem Vektor pUC18)

Die entstandenen DNA-Fragmente wurden durch Agarose-Gelelektrophorese getrennt und aus dem Gel eluiert (s. Beispiel 6). Das Vektorplasmid pUC18 wurde aus E. coli DH5α präpariert , mit HindIII und PstI hydrolysiert und mit alkalischer Phosphatase (Boehringer, Mannheim, Deutschland) nach Vorschrift des Herstellers behandelt. Das 2.6 kb HindIII / PstI-Fragment wurde kloniert. Die Ligation und Transformation wurde wie unter Beispiel 10 beschrieben durchgeführt. Das Plasmid mit dem 2.6 kb HindIII / PstI-Fragment bekam die Bezeichnung pAS5/15.1.

**13. Amplifizierung und Klonierung zweier 0.3 kb DNA-Fragmente aus chromosomaler DNA von Actinoplanes sp.**

Um den überlappenden DNA-Bereich zwischen den in den Plasmiden pAS5/15.5 und pAS5/15.6 klonierten DNA-Abschnitten zu sequenzieren, wurden zwei Primer (acbD3 und acbD4) aus den bekannten DNA-Sequenzen dieser Plasmide (s. Beispiel 14) synthetisiert. Mit Hilfe dieser Primer wurde ein 0.3 kb DNA-Fragment aus chromosomaler DNA von Actinoplanes sp. amplifiziert. Die Denaturierungstemperatur betrug 95°C (1 min), die Annealingtemperatur betrug 68°C (20 sec) und die Primer-Verlängerung erfolgte bei 72°C (20 sec). Es wurden 25 Amplifkationszyklen durchgeführt. Die Taq-Polymerase wurde nach Angaben des Herstellers (Gibco BRL, Eggenstein, Deutschland) eingesetzt. Der PCR-Ansatz enthielt 5 % Formamid. Für die PCR-Reaktion wurde der Personal-Cycler der Firma BIOMETRA (Göttingen, Deutschland) eingesetzt. Der PCR-Ansatz wurde ethanolisch gefällt, anschließend in pUC18 (hydrolysiert mit HindIII) ligiert und in E. coli DH5α kloniert. Um den überlappenden DNA-Bereich zwischen den Plasmiden pAS5/15.4 und pAS5/15.2 zu sequenzieren wurden mittels der Primer acbD5 und acbD6 ein weiteres 0.3 kb DNA-Fragment mit dem gleichen Versuchsansatz amplifiziert und kloniert.

pAS5/15.18, pAS5/15.19. Das PCR-Fragment, das mit den Primern acbD3 und acbD4 amplifiziert wurde; ergab nach der Klonierung den Subklon pAS5/15.18. Das PCR-Fragment, das mit den Primern acbD5 und acbD6 amplifiziert wurde, ergab nach der Klonierung den Subklon pAS5/15.19.

**14. Subklonierung von Fragmenten aus dem Plasmid pAS5**

Ausgehend von dem Plasmid pAS5 wurden mehrere Fragmente subkloniert, um die Sequenz der Doppelstrang-DNA aufzuklären (Abb. 1).

pAS5/15.1 Das Plasmid pAS 5 wurde mit den Restriktionsenzymen HindIII und PstI hydrolysiert. Von den fünf entstandenen Fragmenten (s. Beispiel 12) wurde das 2.6 kb HindIII / PstI-Fragment kloniert. Dazu wurde der Restriktionsansatz auf einem 0.7 %igen Agarosegel getrennt, das 2.6 kb HindIII / PstI-Fragment aus dem Gel eluiert (s. Beispiel 6), in pUC18 (hydrolysiert mit HindIII / PstI) ligiert und in E. coli DH5α kloniert.

pAS5/15.2; pAS5/15.3; pAS5/15.4; pAS5/15.5 Das Plasmid pAS5/15.1 wurde mit dem Restriktionsenzym SalI hydrolysiert. Die dabei entstandenen 6 Fragmente wurden auf einem 1%igen Agarosegel getrennt. Die Fragmente hatten folgende Größen: 0.75 kb, 0.5 kb, 0.4 kb, 0.35 kb, 0.05 kb und 3.2 kb (0.5 kb-Fragment ligiert mit pUC18). Die für die Subklonierung vorgesehenen Fragmente wurden aus dem Gel eluiert (s. Beispiel 6). Für die Klonierung wurde der Vektor pUC18 mittels des Restriktionsenzyms SalI wie unter Beispiel 6 beschrieben präpariert.

Die Ligationen wurden wie unter Beispiel 10 beschrieben durchgeführt. Das 0.75 kb-Fragment wurde in den präparierten pUC18 ligiert und es entstand das Plasmid pAS5/15.2. Das Plasmid pAS5/15.3 entstand nach der Ligation des 0.5 kb-Fragments mit dem präparierten pUC18. Das Plasmid pAS5/15.4 enthält das 0.4 kb-Fragment und das 0.35 kb-Fragmentist Bestandteil des Plasmids pAS5/15.5.

pAS5/15.6; pAS5/15.7; pAS5/15.9 Das Plasmid pAS5/15.1 wurde mit dem Restriktionsenzm PvuII hydrolysiert. Die dabei entstandenen 5 Fragmente wurden auf einem 1.2 %igen Agarosegel getrennt. Die Fragmente hatten folgende Größen:

1.25 kb PvuII-Fragment
0.15 kb PvuII -Fragment
0.8 kb PvuII-Fragment (0.7 kb PvuII / HindIII-Fragment ligiert mit 0.1 kb HindIII / PvuII -Fragment aus pUC18).
0.66 kb PvuII-Fragment (0.5 kb PvuII / PstI -Fragment ligiert mit 0.16 kb PstI / PvuII-Fragment aus pUC18)
2.4 kb PvuII-Fragment (der Rest des Vektors pUC18)

Das 1.25 kb-Fragment wurde in pUC18 (hydrolysiert mit HincII) ligiert und in E. coli DH5α kloniert und es entstand das Plasmid pAS5/15.6. Das Plasmid pAS5/15.7 entstand nach der Klonierung des 0.8 kb-Fragments in das mit HincII hydrolysierte Vektorplasmid pUC18. Das Plasmid pAS5/15.9 enthält das 0.15 kb-Fragment.

<u>pAS5/15.12</u> Das Plasmid pAS5/15.1 wurde mit den Restriktionsendonukleasen NcoI und KpnI hydrolysiert. Das dadurch entstandene 0.9 kb NcoI / KpnI-Fragment wurde aus einem 1.2 %igen Agarosegel eluiert (s. Beispiel 6) und in den Vektor pUCBM21 (hydrolysiert mit NcoI / KpnI) ligiert und in E. coli DH5α kloniert; es entstand das Plasmid

pAS5/15.12.

**15. DNA-Sequenzierung des 2.6 kb HindIII / PstI-Fragments von Actinoplanes sp.**

Es wurden die in den Beispielen 13 und 14 beschriebenen Plasmide sequenziert. In die Sequenzierungsreaktion wurden 6 - 8 μg Plasmid-DNA aus einer Präparation (s. Beispiel 6) eingesetzt. Die Sequenzierreaktion wurde mit dem Auto-Read-Sequenzing-Kit (Pharnmacia, Freiburg, Deutschland) durchgeführt. Hierbei kam das Standardprotokoll zur Sequenzierung von dsDNA zur Anwendung. Um die Auswertung der Nukleotidsequenz mit dem A.L.F. zu ermöglichen, wurden als Startermoleküle für die Sequenzreaktion die Fluorescein-markierten universellen und reversen Sequenzier-Primer verwendet (s. Tab. 3). Zur Herstellung des Gels wurden 8 ml Hydro Link Long Ranger (Serva, Heidelberg, Deutschland), 33.6 g Harnstoff, 8 ml 10x TBE-Puffer, ad 80 ml mit $H_2O$ vermischt, sterilfiltriert und 1 Minute entgast. Die Polymerisation wurde durch Zugabe von 350 μl 10 % (w/v) Ammoniumpersulfät und 40 μl N,N,N',N'-Tetramethylethylendiamin eingeleitet. Die Lösung wurde in eine Gelform (50 x 50 x 0.05 cm) gegossen. Die Elektrophorese erfolgte bei 38 W und einer konstanten Temperatur von 45°C. Als Laufpuffer diente 1 x TBE-Puffer. Die Verarbeitung der gemessenen Fluoreszenz zu einer DNA-Sequenz erfolgte über einen angeschlossenen Computer (Compaq 386/20e), der auch zur Steuerung der Elektrophoreseeinheit diente (Programm A.L.F. Manager 2.5; Pharmacia).

**16. Überexpression der Acarviosyl-Transferase in S. lividans**

Das Gen für die Acarviosyl-Transferase (acbD) aus Actinoplanes sp. wurde in Streptomyces lividans TK21 in dem Shuttle-Vektor pUWL201 (U. Wehmeier, unveröffentlicht, Abb. 2) zur Expression gebracht. Das Plasmid (6.4 kb) setzt sich aus dem Vektor pUWL199 (Wehmeier, 1995) zusammen, in dem das 2.0 kb KpnI / XbaI-Fragment durch ein KpnI / XbaI-Fragment, bestehend aus dem ermE*p-Promotor (Bipp et al., 1994) und dem HincII / ClaI-Anteil des Multilinkers aus pBLUESCRIPT (Stratagene) ersetzt ist. Für die Klonierung des acbD-Gens in E. coli DH5α und Streptomyces lividans TK21 wurde das Plasmid pAS5/15.1 (s. Beispiel 12) mit den Restriktionsendonukleasen HindIII und PstI hydrolysiert. Das dabei entstandene 2.6 kb HindIII / PstI-Fragment wurde in den Vektor pUWL201 (hydrolysiert mit HindIII und PstI) ligiert und in E. coli DH5α kloniert. Das resultierende Plasmid bekam die Bezeichnung pAS9. Das Plasmid pAS9 wurde durch alkalische Lyse aus E. coli DH5α präpariert und mit der Methode der Protoplasten-Transformation (Hopwood et al., 1985) in S. lividans TK21 kloniert. Das acbD-Gen steht in diesem Klon unter der Kontrolle des konstitutiven ermE*p-Promotors (M. Bibb, Norwich, England; persönl. Mitteilung). Nach der Kultivierung von S. lividans TK21/pAS9 in TSB-Medium (25 μg/ml Thiostrepton) konnte im Überstand eine signifikante Bande eines zusätzlichen Proteins von 75 kDa auf einem SDS-Polyacrylamidgel (Lugtenberg et al., 1975) nachgewiesen werden.

**17. Inaktivierung der Acarviosyl-Transferase durch gene-disruption**

Das Gen für die Acarviosyl-Transferase (acbD) aus Actinoplanes sp. wurde durch die Methode des gene-disruption inaktiviert. Zu diesem Zweck wurde das chromosomale acbD-Gen in Actinoplanes sp. teilweise durch ein Antibiotikaresistenzgen ersetzt. Die Antibiotikaresistenzgene wurden durch homologe Rekombination eingeführt. In Vorversuchen konnte gezeigt werden, daß Actinoplanes sp. gegen die Antibiotika Erythromycin, Streptomycin, Apramycin, Neomycin und Kanamycin sensitiv ist. Daher wurden die Antibiotikaresistenzgene ermE, aphD1, aaC4 und aph für die Mutagenese eingesetzt. In einem ersten Beispiel wurde das Erythromycin-Resistenzgen (ermE) aus dem Vektor pUGT1 (Ingham et al., 1995) für die Inaktivierung von acbD genutzt. Dazu wurde das Resistenzgen auf einem 1.5 kb großen KpnI-Fragment nach Hydrolyse von pUGT1 mit KpnI auf einem Agarosegel getrennt und aus dem Gel isoliert. Das Plasmid pAS5/15.1 (s. Beispiel 12) wurde mit der Restriktionsendonuklease NcoI linearisiert. Die NcoI-Erkennungssequenz liegt auf dem klonierten chromosomalen 2.6 kb Fragment bei 1050 bp. Die hydrolysierten Enden des Plasmids pAS5/15.1 und des präparierten 1.5 kb KpnI-Fragments wurden mit dem Klenow-Fragment der DNA-Polymerase I nach Angaben des Herstellers (Gibco BRL, Eggenstein, Deutschland) in glatte DNA-Doppelstrangenden überführt. Durch die anschließende Ligation wurde das Erythromycinresistenzgen, das auf dem 1 5 kb KpnI-Fragment liegt, in das acbD-Gen auf dem Plasmid pAS5/15.1 in E. coli DH5α kloniert. Dieses Plasmid wurde linearisiert und durch übliche Methoden (Protoplastentransformation) eingebracht. Auch durch Konjugation mit E. coli S17-1 und Actinoplanes sp. kann das rekombinante Plasmid übertragen wereen. Eine zusätzliche Möglichkeit der Plasmid-Übertragung stellt die Methode der Elektroporation dar. Durch homologe Rekombination wurde das chromosomale acbD-Gen gegen das durch das Erythromycinresistenzgen unterbrochene acbD-Gen des konstruierten Plasmids ausgetauscht. Durch ein Doppel-Crossover entstand eine Erythromycin-resistente acbD-Mutante von Actinoplanes sp. SE 50/110. Für die Einrekombination alternativer Resistenzgene in Actinoplanes sp. können ebenfalls folgende Methoden angewandt werden: (1) Elektroporation, (2) Protoplasten-Transformation (Hopwood et al., 1985), (3) Transformation von Mycel (Madon u. Hutter, 1991) und (4) Konjugation (Mazodier et al., 1989).

Puffer und Lösungen:

Medien für die Bakterienanzucht

LB-Medium:

| | |
|---|---|
| Trypton | 10 g |
| NaCl | 10 g |
| Hefeextrakt | 5 g |
| $H_2O$ | ad 1000 ml |

Es wurde mit 4 M NaOH ein pH-Wert von 7.5 eingestellt

TSB-Medium:

| | |
|---|---|
| Tryptone-Soya Broth (Oxoid) | 30 g |
| $H_2O$ | ad 1000 ml |

TE-Puffer (pH 8.0)

| | |
|---|---|
| Tris-HCl | 10 mM |
| $Na_2$-EDTA | 1 mM |

Standardpräparation von Plasmid-DNA
(modif. nach Birnboim u. Doly, 1979)

| | |
|---|---|
| Mix I | 50 mM Glucose<br>50 mM Tris-HCl (pH 8.0)<br>10 mM EDTA (pH 8.0)<br>5 mg/ml Lysozym |
| Mix II | 200 ml NaOH<br>1 % (w/v) SDS (Natriumdodecylsulfat) |
| Mix III | 3 M Kaliumacetat<br>1.8 M Formiat |

DNA-DNA-Hybridisierung

| | |
|---|---|
| 20x SSC | 3 M NaCl<br>0.3 M Na-Citrat |

Prähybridisierungslösung:

6 x SSC
0.01 M Natriumphosphatpuffer pH 6.8
1 mM EDTA

0.5 % SDS
0.1 % Magermilchpulver

Hybridisierungslösung:
In 15 ml Prähybridisierungslösung wird die acb-Sonde nach der Markierungsreaktion gegeben.

6x Postwash
6x SSC
0.5 % SDS

DNA-Sequenzierung
TBE-Puffer (pH 8.0)
1 M Tris-Base
0.83 M Borsäure
10 mM EDTA

**Literatur:**


1) Bibb, M.J. et al. (1994)
The mRNA for the 23S rRNA methylase encoded by the ermE gene of Saccharopolyspora erythraea is translated in the absence of a conventional ribosome-binding site
Mol. Microbiol. 14, 533-545.

2) Birnboim, H.C., J. Doly (1979)
A rapid alkaline extraction procedure for screening recombinant plasmid DNA
Nucleic Acids Res. 7, 1513-1523

3) Hanahan, D. (1983)
Studies on transformation of Escherichia coli with plasmids
J. Mol. Biol. 166, 557-580

4) Hopwood, D.A. et al. (1985)
Genetic manipulation of Streptomyces;
A laboratory manual; The John Innes Foundation, Norwich, England

5) Ingham, C.J., et al. (1995)
Rho-independent terminators without 3'poly-U tails from the early region of actinophage phi C31
Nucleic Acids Res. 23, 370-373.

6) Lugtenberg, B. et al. (1975)
Electrophoretic resolution of the major outer membrane protein of Escherichia coli into four bands
FEBS Lett. 58, 254-258.

7) Madon, J., R. Hütter (1991)
Transformation System for Amycolatopsis (Nocardia) mediterranei; direct transformation of mycelium with plasmid DNA.

J. Bacteriol. 173, 6325-6331

8) Mazodier, P. et al. (1989)
Intergenic conjugation between Escherichia coli and Streptomyces Species
J. Bacteriol. 171, 3583-3585

9) Sanger, F. et al. (1977)
DNA sequencing with chain terminating inhibitors
Proc. Natl. Acad. Sci. USA 74, 5463-5467

10) Southern, E.M. (1975)
Detection of specific sequences among DNA fragments separated by gel electrophoresis
J. Mol. Biol. 98, 503-521

11) Wehmeier, U.F. (1995)
New functional Escherichia coli-Streptomyces shuttle vectors allowing blue- white screening on Xgal plates
Gene 165, 149-150

(1) ALLGEMEINE ANGABEN:

(i) ANMELDER:
(A) NAME: Bayer AG
(B) STRASSE: Bayerwerk
(C) ORT: Leverkusen
(E) LAND: Germany
(F) POSTLEITZAHL: 51368
(G) TELEFON: 0214-3061455
(H) TELEFAX: 0214-303482

(ii) BEZEICHNUNG DER ERFINDUNG: Verfahren zur Herstellung sowie Verwendung von Acarviosyltransferase bei der Umwandlung von Acarbose-Homologen in Acarbose, zur Herstellung von Acarbosehomologen

(iii) ANZAHL DER SEQUENZEN: 16

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30B (EPA)

(v) DATEN DER JETZIGER ANMELDUNG:
ANMELDENUMMER: EP 97104115

(2) ANGABEN ZU SEQ ID NO: 1:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 1:

ACCAGGCCGA GGACGGCGCC C 21

(2) ANGABEN ZU SEQ ID NO: 2:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 2:

AGCGGCATGT GCTTGACGGC G 21

(2) ANGABEN ZU SEQ ID NO: 3:

(i) SEQUENZKENNZEICHEN:
(A) LÄNGE: 23 Basenpaare
(B) ART: Nucleotid
(C) STRANGFORM: Einzelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 3:

ACCGGCTCGA ACGGGCTGGC ACC 23

(2) ANGABEN ZU SEQ ID NO: 4:

(i) SEQUENZKENNZEICHEN:
(A) LÄNGE: 21 Basenpaare
(B) ART: Nucleotid
(C) STRANGFORM: Einzelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 4:

CCCTCGACGG TGACGGTGGC G 21

(2) ANGABEN ZU SEQ ID NO: 5:

(i) SEQUENZKENNZEICHEN:
(A) LÄNGE: 17 Basenpaare
(B) ART: Nucleotid
(C) STRANGFORM: Einzelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 5:

GTAAAACGAC GGCCAGT 17

(2) ANGABEN ZU SEQ ID NO: 6:

(i) SEQUENZKENNZEICHEN:
(A) LÄNGE: 18 Basenpaare
(B) ART: Nucleotid
(C) STRANGFORM: Einzelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 6:

GAAACAGCTA TGACCATG 18

(2) ANGABEN ZU SEQ ID NO: 7:

(i) SEQUENZKENNZEICHEN:
(A) LÄNGE: 23 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 7:

```
Asn Leu Gly Val Gly Ala Ile Trp Ile Ser Pro His Val Asp Asn Ile
1               5                   10                  15

Asn Val Pro Ala Ala Gly Gly
            20
```

(2) ANGABEN ZU SEQ ID NO: 8:

(i) SEQUENZKENNZEICHEN:
(A) LÄNGE: 21 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 8:

```
Thr Gly Lys Pro Val Pro Val Gln Phe Thr Val Gln Asn Pro Pro Ala
1               5                   10                  15

Thr Ala Pro Gly Glu
            20
```

(2) ANGABEN ZU SEQ ID NO: 9:

(i) SEQUENZKENNZEICHEN:
(A) LÄNGE: 18 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 9:

```
Ser Thr Val Ala Pro Val Leu Gly Ala Gly Gln Val Ala Val Trp Ser
1               5                   10                  15

Tyr Arg
```

(2) ANGABEN ZU SEQ ID NO: 10:

(i) SEQUENZKENNZEICHEN:
(A) LÄNGE: 20 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 10:

```
Tyr Gln Asp Gln Tyr Tyr Ser Leu Ala Asp Ile Ala Asp Leu Asp Gln
1               5                   10                  15

Gln Asn Pro Arg
            20
```

(2) ANGABEN ZU SEQ ID NO: 11:

(i) SEQUENZKENNZEICHEN:
(A) LÄNGE: 12 Aminosäuren
(B) ART: Aminosäure
(C) STRANGFORM: Einzelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 11:

```
Trp Ile Asn Asp Asp Val Tyr Val Tyr Glu Arg Leu
1               5                   10
```

(2) ANGABEN ZU SEQ ID NO: 12:

(i) SEQUENZKENNZEICHEN:
(A) LÄNGE: 18 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 12:

```
Asp Tyr Leu Tyr Glu Gln Asp Leu Ile Thr Phe Leu Asp Asn Gln Asp
1               5                   10                  15

Thr Arg
```

(2) ANGABEN ZU SEQ ID NO: 13:

(i) SEQUENZKENNZEICHEN:
(A) LÄNGE: 9 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 13:

```
Asp Asp Ala Asn Tyr Trp Met Asp Arg
1               5
```

(2) ANGABEN ZU SEQ ID NO: 14:

(i) SEQUENZKENNZEICHEN:
(A) LÄNGE: 12 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 14:

```
Ala Val Leu Thr Gly Asn Thr Val Tyr Asp Trp Lys
1               5                   10
```

(2) ANGABEN ZU SEQ ID NO: 15:

(i) SEQUENZKENNZEICHEN:
(A) LÄNGE: 2582 Basenpaare
(B) ART: Nucleotid
(C) STRANGFORM: Einzelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 15:

```
AAGCTTGAAG TGGCGGTGAT GCATCCATCA CTGTATGCGC ATCTGAATGA CGTCTTCTGC      60
AAGTTCTTGC AGCGGTCTCC GGGCCCTGCC CTTCCTCGTC ATCCCTTCAC AAGGAGAAGC     120
TCGTGCAACG TCACGCCAGG CATGCCATCG CCGCGGCGGT AGGCTTTCCG CTGCTGCCGC     180
CGTCACTGCC GGCTCATGCC GCCGGGGCTT CGGCCGTGGT GCCGTACGCC GGTAACCCGG     240
CCAGTCTCAA GCAGGACCTC TGCTACCAGA TCGCCACCGA CCGGTTCAGC GACGGGACCC     300
CGGCGAACAA CAATCCGGGC AACGTGCCCG GCATGTTCGC CGACAAGACC AAGCTGAACG     360
ACCGGCAGGA GTGGCTCAAA TACATGGGAG GTGACTTCGC CGGCATCACC CAGCGGATGG     420
AGTACCTCAA GAACCTGGGC GTCGGCGCGA TCTGGATCTC GCCGCACGTC GACAACATCA     480
ACGTTCCGGC GAACGGCGCC ACCGGTTACC ACGGCTACTG GCCGCGCGAC TTCAAGCGGC     540
TCGAAGAGCA CTTCGGCACC GACGAGGAGT TCGACGCGCT GGTGTCGGCG GCGCACGCCA     600
GCAACATCAA AGTGATCATG GACTGGACGC CGAACGGCAC CAACCCGCCG AACCAGGCCG     660
AGGACGGCGC CCTCTACGAC GATGGGCAGC TGGTCGGCAG GTACGGGGCG GACAGTGCCG     720
GGCACTTCCA CCACGGCCCG GCGATCGGCG ACTTCAACGA TCGCTACCAG GACCAGTACT     780
ACAGCCTGGC CGACATCGCC GACCTCGACC AGCAGAACCC GCGGGTCGAC CAGCTGCTCA     840
AGGACGACGC CAACTACTGG ATGGACCGCG GGGTCGACGG CATCCGGGTC GACGCCGTCA     900
AGCACATGCC GCTGAGCTGG CAGCGGTCCT TCGCCGACGC GGTCACCTCG CACAAGAGCG     960
CGGCCATCTT CGGCGAGTGG TACATGGGCG ACCAGTCCGA TCCGCTCTAC GCCGACCAGG    1020
TCAAGTTCGC CAACACCAGC GGCATCGCGG CCATGGACTT CTACACCAAC CGCTCGATCC    1080
GCGACACCTT CGCCGGCGCC GGCTCGATGA AGTCCCTGGA CGCGGCGATC ACCAAGACCA    1140
ACCGGGACTA CCTCTACGAG CAGGATCTGA TCACGTTCCT GGACAACCAG GACACCCGGC    1200
GCTTCGGGAC GCTCAACAGC GATCCGGCGG CCCTGCACCG GGCGCTCGCC TTCCTGCTCA    1260
CCACCCGGGG TACGCCGTGC CTGTTCTACG GCACCGAGCA GTACCTGCAC AACGACACCG    1320
GTGAGGGCAG CAACAAGGGC AAGGACCCGT ACAACCGGCC CCCGATGGCC AGTTTCGACA    1380
CCGACACGGT CGCCTACCGG GAGATCCGGC GCCCTCTCCG ACCTGCGCCG GTCGAACCCC    1440
GCGGTGGCTA CGGGGACCAC CAGCAGCGGT GGATCAACGA CGACGTGTAC GTCTACGAGC    1500
GCCGGTTCGG CGACAACGTG CTGCTGACCG CCATCAACAA GGGCTCGCAC GAGTACCGGC    1560
TCGAACGGGC TGGCACCGCG CTGCCGGCCG GCACCTATCG CGACGTGCTC GGCGGCACCT    1620
TCGGCGGCTC CGACCTGACC GTCGAGGACG GCGACGGCAC CGACCGGTCG ACCGTCGCGC    1680
CGGTGCTGGG TGCCGGGCAG GTCGCCGTCT GGTCGTACCG GGCGCCGGTG GACACCGAGC    1740
CCCGGATCGG CGGGGTCGGG CCGGTCGTGA CCCGGGCCGG CGCCACCGTC ACCGTCGAGG    1800
GCACCGGCTT CGGCTCCGGC GGAACCGTCG CGATCGGCGG AGTCCCCGCG ACCGTCCAGC    1860
```

AGTGGACGGC GGACCGTATC ACCGCCACCG TCCCGGTCGG CGTTCCCACC GGGGCCGTCC 1920

AGGTGACCGT CGGCAACGGC TCCGGCACCA GCAACGGGTA CCCGATCACC ACCCGTACCG 1980

GAAAACCGGT CCCGGTGCAG TTCACCGTTC AGAACCCGCC GGCCACCGCG CCCGGGGAGT 2040

CGCTCTACCT GACCGGTGAC GTCGCCGAGT TGGGGCACTG GTCGACCAGC CCGGACCAGA 2100

CCGCGGGACA GCTGCTGCGG GTGCCGAACG AGTCCCGGGG CGTCCTCGTC GCCGACCTGC 2160

CGGCCGGGGC GCCGGTCGAG TTCAAGTTCG TCAAGGTCGC GGCCGACGGC ACGGTGACCT 2220

GGGAGGGTGG TGCCAACCAC CGGTACACCG TCCCGGCCGG CGGCACCGGC ACGACCAGCC 2280

TCACCTGGCA GCGCTGACGC CACCGTGCGG AGGGCCCGGC CGTGACCGGG CCCGCCGCAC 2340

CGGGCCGGGC GGTGGAACGG CCGGGACGGT TGGGCGCCGG CCCCGGCGTG CGCAGATCGA 2400

GGGCTGCGCA CACCGGGGGC TTGAACGGCT GGTCTGGCCC CAAGGCGACG GTTCCCGTCG 2460

GCGAGTATCT CACCTTCAAG GGTCCCCGGA CGCCTCGCCC GGCTTCTCCA CCAGCGCCGA 2520

CGGTTACCAG ATCACCCCTT GGTGGAGCCG AGGGAGAGGC CGGCGATGAA CTGCTTCTGC 2580

AG 2582

(2) ANGABEN ZU SEQ ID NO: 16:

(i) SEQUENZKENNZEICHEN:
(A) LÄNGE: 725 Aminosäuren
(B) ART: Aminosäure
(C) STRANGFORM: Einzelstrang
(D) TOPOLOGIE: linear

(ii) ART DES MOLEKÜLS: Genom-DNA

(iii) HYPOTHETISCH: NEIN

(iv) ANTISENSE: NEIN

(vi) URSPRÜNLICHE HERKUNFT:
(A) ORGANISMUS: Actinoplanes

(xi) SEQUENZBESCHREIBUNG: SEQ ID NO: 16:

Val Gln Arg His Ala Arg His Ala Ile Ala Ala Ala Val Gly Phe Pro
1 5 10 15

Leu Leu Pro Pro Ser Leu Pro Ala His Ala Ala Gly Ala Ser Ala Val
20 25 30

Val Pro Tyr Ala Gly Asn Pro Ala Ser Leu Lys Gln Asp Leu Cys Tyr
35 40 45

Gln Ile Ala Thr Asp Arg Phe Ser Asp Gly Thr Pro Ala Asn Asn Asn
50 55 60

Pro Gly Asn Val Pro Gly Met Phe Ala Asp Lys Thr Lys Leu Asn Asp
65 70 75 80

Arg Gln Glu Trp Leu Lys Tyr Met Gly Gly Asp Phe Ala Gly Ile Thr
85 90 95

```
Gln Arg Met Glu Tyr Leu Lys Asn Leu Gly Val Gly Ala Ile Trp Ile
            100                 105                 110

Ser Pro His Val Asp Asn Ile Asn Val Pro Ala Asn Gly Ala Thr Gly
        115                 120                 125

Tyr His Gly Tyr Trp Pro Arg Asp Phe Lys Arg Leu Glu Glu His Phe
    130                 135                 140

Gly Thr Asp Glu Glu Phe Asp Ala Leu Val Ser Ala Ala His Ala Ser
145                 150                 155                 160

Asn Ile Lys Val Ile Met Asp Trp Thr Pro Asn Gly Thr Asn Pro Pro
                165                 170                 175

Asn Gln Ala Glu Asp Gly Ala Leu Tyr Asp Asp Gly Gln Leu Val Gly
            180                 185                 190

Arg Tyr Gly Ala Asp Ser Ala Gly His Phe His His Gly Pro Ala Ile
        195                 200                 205

Gly Asp Phe Asn Asp Arg Tyr Gln Asp Gln Tyr Tyr Ser Leu Ala Asp
    210                 215                 220

Ile Ala Asp Leu Asp Gln Gln Asn Pro Arg Val Asp Gln Leu Leu Lys
225                 230                 235                 240

Asp Asp Ala Asn Tyr Trp Met Asp Arg Gly Val Asp Gly Ile Arg Val
                245                 250                 255

Asp Ala Val Lys His Met Pro Leu Ser Trp Gln Arg Ser Phe Ala Asp
            260                 265                 270

Ala Val Thr Ser His Lys Ser Ala Ala Ile Phe Gly Glu Trp Tyr Met
        275                 280                 285

Gly Asp Gln Ser Asp Pro Leu Tyr Ala Asp Gln Val Lys Phe Ala Asn
    290                 295                 300

Thr Ser Gly Ile Ala Ala Met Asp Phe Tyr Thr Asn Arg Ser Ile Arg
305                 310                 315                 320

Asp Thr Phe Ala Gly Ala Gly Ser Met Lys Ser Leu Asp Ala Ala Ile
                325                 330                 335

Thr Lys Thr Asn Arg Asp Tyr Leu Tyr Glu Gln Asp Leu Ile Thr Phe
            340                 345                 350

Leu Asp Asn Gln Asp Thr Arg Arg Phe Gly Thr Leu Asn Ser Asp Pro
        355                 360                 365

Ala Ala Leu His Arg Ala Leu Ala Phe Leu Leu Thr Thr Arg Gly Thr
    370                 375                 380

Pro Cys Leu Phe Tyr Gly Thr Glu Gln Tyr Leu His Asn Asp Thr Gly
385                 390                 395                 400

Glu Gly Ser Asn Lys Gly Lys Asp Pro Tyr Asn Arg Pro Pro Met Ala
                405                 410                 415

Ser Phe Asp Thr Asp Thr Val Ala Tyr Arg Glu Ile Arg Arg Pro Leu
            420                 425                 430

Arg Pro Ala Pro Val Glu Pro Arg Gly Gly Tyr Gly Asp His Gln Gln
        435                 440                 445
```

```
Arg Trp Ile Asn Asp Asp Val Tyr Val Tyr Glu Arg Arg Phe Gly Asp
    450                 455                 460

Asn Val Leu Leu Thr Ala Ile Asn Lys Gly Ser His Glu Tyr Arg Leu
465                 470                 475                 480

Glu Arg Ala Gly Thr Ala Leu Pro Ala Gly Thr Tyr Arg Asp Val Leu
                485                 490                 495

Gly Gly Thr Phe Gly Gly Ser Asp Leu Thr Val Glu Asp Gly Asp Gly
            500                 505                 510

Thr Asp Arg Ser Thr Val Ala Pro Val Leu Gly Ala Gly Gln Val Ala
        515                 520                 525

Val Trp Ser Tyr Arg Ala Pro Val Asp Thr Glu Pro Arg Ile Gly Gly
    530                 535                 540

Val Gly Pro Val Val Thr Arg Ala Gly Ala Thr Val Thr Val Glu Gly
545                 550                 555                 560

Thr Gly Phe Gly Ser Gly Gly Thr Val Ala Ile Gly Gly Val Pro Ala
                565                 570                 575

Thr Val Gln Gln Trp Thr Ala Asp Arg Ile Thr Ala Thr Val Pro Val
            580                 585                 590

Gly Val Pro Thr Gly Ala Val Gln Val Thr Val Gly Asn Gly Ser Gly
        595                 600                 605

Thr Ser Asn Gly Tyr Pro Ile Thr Thr Arg Thr Gly Lys Pro Val Pro
    610                 615                 620

Val Gln Phe Thr Val Gln Asn Pro Pro Ala Thr Ala Pro Gly Glu Ser
625                 630                 635                 640

Leu Tyr Leu Thr Gly Asp Val Ala Glu Leu Gly His Trp Ser Thr Ser
                645                 650                 655

Pro Asp Gln Thr Ala Gly Gln Leu Leu Arg Val Pro Asn Glu Ser Arg
            660                 665                 670

Gly Val Leu Val Ala Asp Leu Pro Ala Gly Ala Pro Val Glu Phe Lys
        675                 680                 685

Phe Val Lys Val Ala Ala Asp Gly Thr Val Thr Trp Glu Gly Gly Ala
    690                 695                 700

Asn His Arg Tyr Thr Val Pro Ala Gly Gly Thr Gly Thr Thr Ser Leu
705                 710                 715                 720

Thr Trp Gln Arg Glx
                725
```

**Patentansprüche**

1. Acarviosyl-Transferase.

2. Acarviosyl-Transferase aus Actinoplanes sp. SE 50 oder SE 50/13 oder SE 50/110 und Mutanten.

3. Im wesentlichen reine Acarviosyl-Transferase.

4. Acarviosyl-Transferase mit der Aminosäuresequenz der Abb. 3 und deren funktionellen Derivate.

5. DNA-Sequenz der Abb. 4 codierend für die Acarviosyl-Transferase und deren funktionellen Derivate.

6. Verfahren zur Umwandlung von Acarbose-Nebenkomponenten in Acarbose

$$\text{Acarviosyl-}G_n + \text{Maltose} \rightarrow \text{Acarbose} + G_n$$

worin

$G_n$ für Glucose, Disaccharide und Maltooligosaccharide steht, dadurch gekennzeichnet, daß die Reaktion durch eine Acarviosyl-Transferase katalysiert wird.

7. Verwendung von Acarviosyl-Transferase bei der Umsetzung von anderen Pseudooligosacchardien wie z. B. Trestatin oder Amylostatin zu Acarbose oder von anderen Glucosidase-Inhibitoren.

8. Verwendung der Acarviosyl-Transferase zur Anreicherung von Acarbose aus Kulturüberständen mit gleichzeitiger Umwandlung von Acarbose-Homologen in Acarbose durch Umsetzung mit hochmolekularen Dextrinen oder Stärke in Gegenwart von Acarviosyl-Transferase

$$\text{Acarviosyl-}(G)_n + (G)_m \rightarrow \text{Acarviosyl-}(G)_m + (G)_n$$

worin G für Glucose steht und m und n eine ganze Zahl zwischen 1 und 20 bedeuten, wobei m und n jeweils unterschiedlich sind,
und Entfernen niedermolekularer Begleitstoffe durch Dialyse, Umkehrosmose oder durch Fällen des Polysaccharids und anschließender Umsetzung mit Maltose unter Freisetzung von Acarbose

$$\text{Acarviosyl-}(G)_m + \text{Maltose} \rightarrow \text{Acarbose} + (G)_m$$

in Gegenwart ausreichend hoher Konzentrationen an Maltose durch Acarviosyl-Transferase.

9. Gentechnische Herstellung der Acarviosyl-Transferase in einem heterologen Wirtsorganismus.

10. Vektor enthaltend die DNA gemäß Anspruch 5.

**Fig. 1**

Plasmidderivate von pAS5 und pAS5/15.1 für die DNA-Sequenzierung

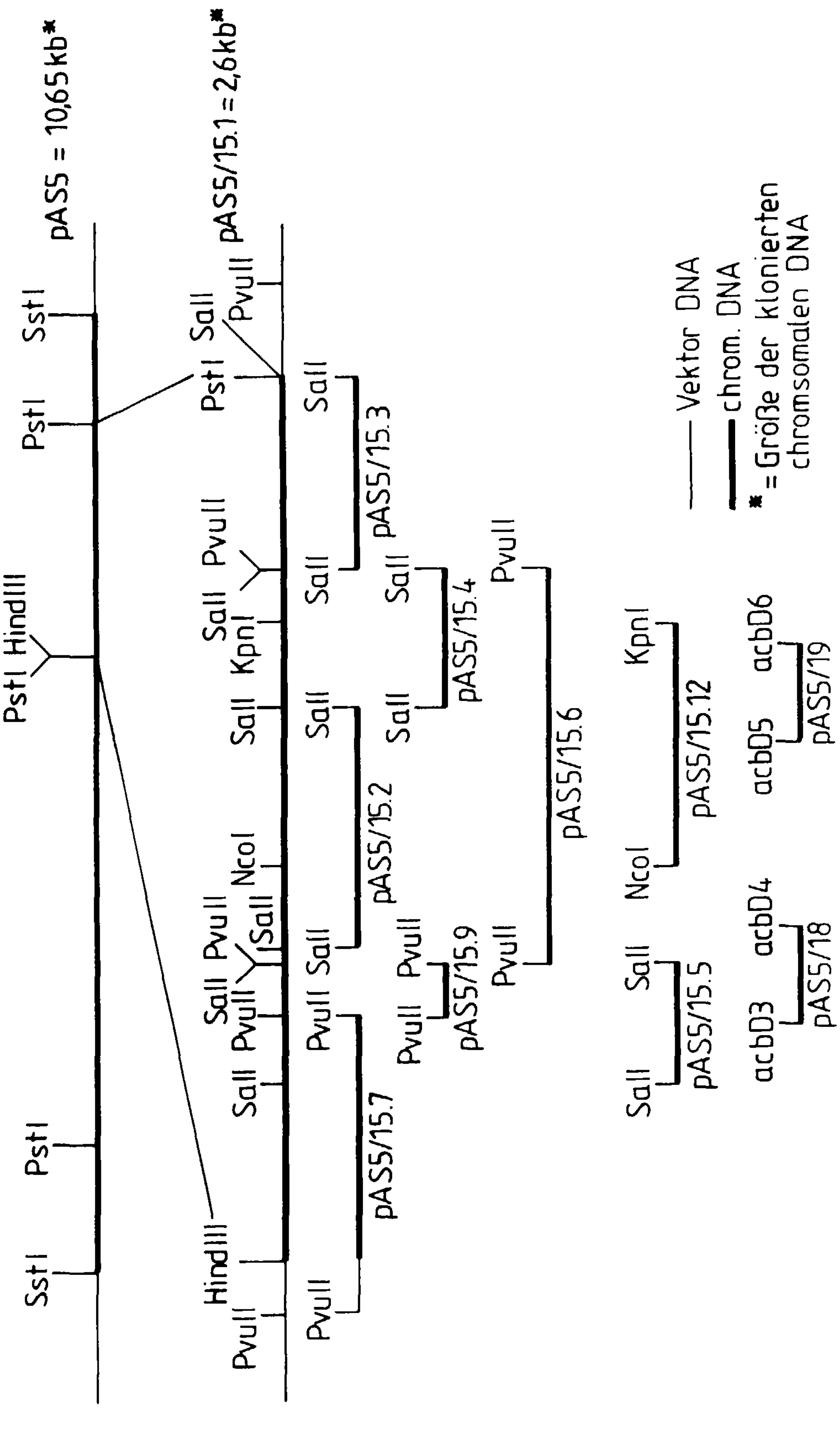

# Fig. 2

Struktur des Expressions-Vektor pUWL201
(U. Wehmeier, unveröffentlicht)

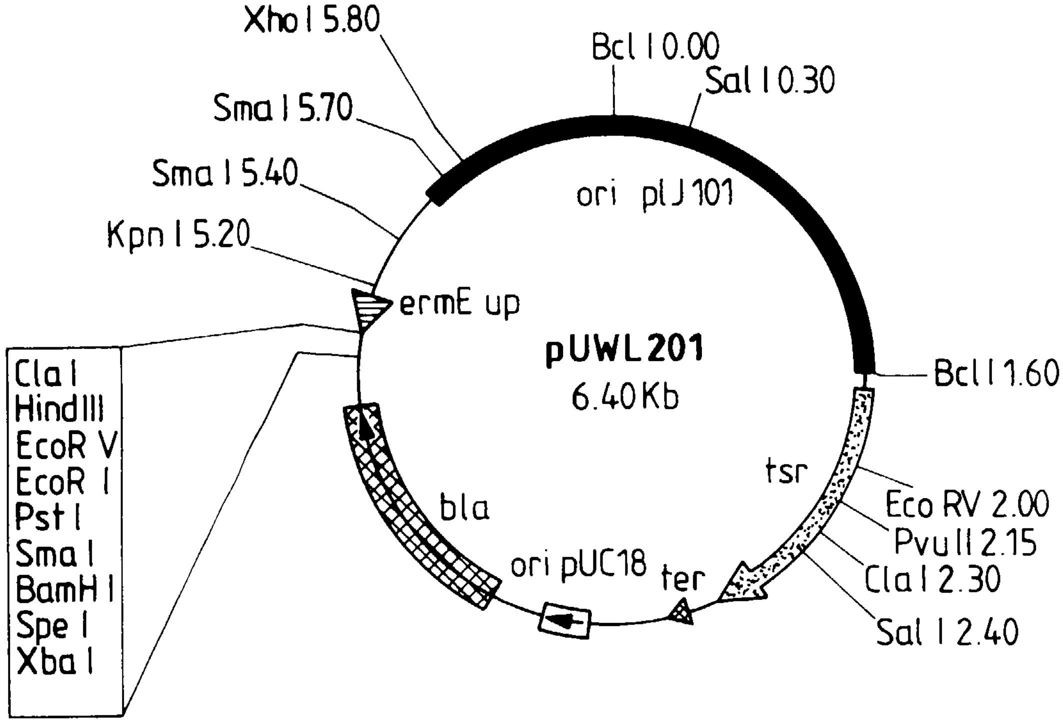

# Fig. 3

Protein sequence 725 a.a. VQRHARHAIAAA ... GTGTTSLTWQRZ

```
        |  10      |  20      |  30      |  40      |  50      |  60
  1 VQRHARHAIA AAVGFPLLPP SLPAHAAGAS AVVPYAGNPA SLKQDLCYQI ATDRFSDGTP 60
 61 ANNNPGNVPG MFADKTKLND RQEWLKYMGG DFAGITQRME YLKNLGVGAI WISPHVDNIN 120
121 VPANGATGYH GYWPRDFKRL EEHFGTDEEF DALVSAAHAS NIKVIMDWTP NGTNPPNQAE 180
181 DGALYDDGQL VGRYGADSAG HFHHGPAIGD FNDRYQDQYY SLADIADLDQ QNPRVDQLLK 240
241 DDANYWMDRG VDGIRVDAVK HMPLSWQRSF ADAVTSHKSA AIFGEWYMGD QSDPLYADQV 300
301 KFANTSGIAA MDFYTNRSIR DTFAGAGSMK SLDAAITKTN RDYLYEQDLI TFLDNQDTRR 360
361 FGTLNSDPAA LHRALAFLLT TRGTPCLFYG TEQYLHNDTG EGSNKGKDPY NRPPMASFDT 420
421 DTVAYREIRR PLRPAPVEPR GGYGDHQQRW INDDVYVYER RFGDNVLLTA INKGSHEYRL 480
481 ERAGTALPAG TYRDVLGGTF GGSDLTVEDG DGTDRSTVAP VLGAGQVAVW SYRAPVDTEP 540
541 RIGGVGPVVT RAGATVTVEG TGFGSGGTVA IGGVPATVQQ WTADRITATV PVGVPTGAVQ 600
601 VTVGNGSGTS NGYPITTRTG KPVPVQFTVQ NPPATAPGES LYLTGDVAEL GHWSTSPDQT 660
661 AGQLLRVPNE SRGVLVADLP AGAPVEFKFV KVAADGTVTW EGGANHRYTV PAGGTGTTSL 720
721 TWQRZ                                                            725
        |  10      |  20      |  30      |  40      |  50      |  60
```

# Fig. 4

DNA sequence 2582 b.p. AAGCTTgaaGTG ... CTGCTTCTGCAG linear

```
           |    10     |    20     |    30     |    40     |    50     |    60
   1 AAGCTTgaaG TGGCGGTGAT GCATCCATCA CTGTATGCGC ATCTGAATGA CGTCTTCTGC 60
  61 AAGTTCTTGC AGCGGTCTCC GGGCCCTGCC CTTCCTCGTC ATCCCTTCAC AAGGAGAAGC 120
 121 TCGTGCAACG TCACGCCAGG CATGCCATCG CCGCGGcGGT AGgcTTTccG CTGCTGCCGC 180
 181 CGTCACTGCC GGCTCATGCC GCCGGGGCTT CGGCCGTGGT GCCGTACgCC GGTAACCCGG 240
 241 CCAGTCTCAA GCAGGACCTC TGCTACCAGA TCGCCACCGA CCGGTTCAGC GACGGGAcCC 300
 301 cggCGAaCAa CAaTCCGGGC AACGTGCCCG gCATGTTCGC CGACAAGACC AAGCTGAACG 360
 361 ACCGGCAGgA GTGGCTCAAA TACATGGGAG GTGACTTCGC CGGCATCACC CAGCGGATGG 420
 421 AGTACCTCAA GAACCTGGGC GTCGGcgCGA TCTGGAtctc gCCGCACGTC GACAACATCA 480
 481 ACGTTCCGGC GAACGGCGCC ACCGGTTACC ACGGCTACTG GCCGCGCGAC TTCAAGCGGC 540
 541 TCGAAGAGCA CTTCGGCACC GACGAGGAGT TCGACGCGCT GGTGTCGGCG GCGCACGCCA 600
 601 GCAACATCAA AGTGATCATG GACTGGACGC CGAACGGCAC CAACCCGCCG AACCAGGCCG 660
 661 AGGACGGCGC CCTcTACGAC GATGGGCAGC TGGTCGGCAG GTACGGGgcg GACAGTGCCG 720
 721 GGCAcTTCCA CCACGGCCCG GCGATCGGCG AcTTCAACGA TCGcTACCAG GACCAgTAcT 780
 781 ACAGcCTGgC CGAcATCGCC GAcCTCGACC AGcagaaccc gcgggtcgac cagCTGCTCA 840
 841 AGGACGACGC CAACTACTGG ATGGACCGCG GGGTCGACGG CATCCGGGTC GACGCCGTCA 900
 901 AGCACATGCC GCTGAGCTGG CAGCGGTCCT TCGCCGACGC GGTCACCTCG CACAAGAGCG 960
 961 CGGCCATCTT CGGCGAGTGG TACATGGGCG ACCAGTCCGA TCCGCTCTAC GCCGACCAGG 1020
1021 TCAAGTTCGC CAACACCAGC GGCATCGCGG CCATGGACTT CTACACCAAC CGCTCGATCC 1080
1081 GCGACACCTT CGCCGGCGCC GGCTCGATGA AGTCCCTGGA CGCGGCGATC ACCAAGACCA 1140
1141 ACCGGGACTA CCTCTACGAG CAGGATCTGA TCACGTTCCT GGACAACCAG GACACCCGGC 1200
1201 GCTTCGGGAC GCTCAACAGC GATCCGGCGG CCCTGCACCG GGCGCTCGCC TTCCTGCTCA 1260
1261 CCACCCGGGG TACGCCGTGC CTGTTCTACG GCACCGAGCA GTACCTGCAC AACGACACCG 1320
1321 GTGAGGGCAG CAACAAGGGC AAGGACCCGT ACAACCGGCC CCCGATGGCC AGTTTCGACA 1380
1381 CCGACACGGT CGCCTACCGG GAGATcCGGC GCCCTCTCCG ACCTGCGCCG GTCGAACCCC 1440
1441 GCGGTGGCTA CGGGGACCAC CAGCAGCGGT GGATCAACGA CGACGTGTAC GTCTACGAGC 1500
1501 gCcGGTTCGG CGACAACGTG CTGCTGACCG CCATCAACAA GGGCTCGCAC GAGTACCGGC 1560
1561 TCGAACGGGC TGGCACCGCG CTGCCGGCCG GCACCTATCG CGACGTGCTC GGCGGCACcT 1620
1621 TCGGCGGCTC CGACCTGACC GTCGAGGACG GCGACGGCAC CGACCGGTCG ACCGTCGCGC 1680
1681 CGGTGCTGGG TGCCGGGCAG GTCGCCGTCT GGTCGTACCG GGCGCCGGTG GACACCGAGC 1740
1741 CCCGGATCGG CGGGGTCGGG CCGGTCGTGA CCCGGGCCGG CGCCACCGTC ACCGTCGAGG 1800
1801 GCACCGGCTT CGGCTCCGGC GGAACCGTCG CGATCGGCGG AGTCCCCGCG ACCGTCCAGC 1860
1861 AGTGGACGGC GGACCGTATC ACCGCCAcCG TCCCGGTCGG CGTTCCCACC GGGGCCGTCC 1920
1921 AGGTGACCGT CGGCAACGGC TCCGGCACCA GCAACGgGTA CCCGATCACC ACcCGTACCG 1980
1981 GAAAACCGGT CCCGGTGCAG TTCACCGTTC AGAACCCGCC GGCCACCGCG CCCGGGGAGT 2040
2041 CGCTCTACCT GACCGGTGAC GTCGCCGAGT TGGGGCACTG GTCGACCAGC CCGGACCAGA 2100
2101 CCGCGGGACA GCTGCTGCGG GTGCCGAACG AGTCCCGGGG CGTCCTCGTC GCCGACCTGC 2160
2161 CGGCCGGGGC GCCGGTCGAG TTCAAGTTCG TCAAGGTCGC GGCCGACGGC ACGGTGACCT 2220
2221 GGGAGGGTGG TGCCAAcCAC cGGTACACCG TCCCGGccGG CGGCACCGGc ACGACCAgcC 2280
2281 TCACCTGGCA GcGCTGACGC CACCGTgcGG AGGgCCCGGC CGTGACCGGG CCCGCCGCAC 2340
2341 CGGGCCGGGC GgTGGAACGG CCGGGACGGT TGGGCGCCGG CCCCGGcGTG CGCAgATCGA 2400
2401 GGGcTGCGCA CACCGGGGGC TTGAACGGCT GGTCTGGCCC CaAGgcGaCG GtTCCCGTCG 2460
2461 GCGAGTATCT CACCTTCAAG GGTCCCCGGA CGCCTCGCCC GGCTTCTCCA CCAGCGCCGA 2520
2521 CGGTTACCAG ATCACCCCTT GGTGGAGCCG AGGCAGAGGC CGGCGATGAA CTGCTTCTGC 2580
                                                                   2582
2581 AG
           |    10     |    20     |    30     |    40     |    50     |    60
```